# EUROPEAN PATENT SPECIFICATION

(11) **EP 0 478 686 B1**
(45) Date of publication and mention of the grant of the patent: **11.08.1993**
(21) Application number: 90910640.3
(22) Date of filing: 21.06.1990
(51) Int. Cl.: C07F 9/10, A61K 31/675, C07F 9/09, C07F 9/6533

(54) **FLUORINE AND PHOSPHOROUS-CONTAINING AMPHIPHILIC MOLECULES WITH SURFACTANT PROPERTIES**
FLUOR- UND PHOSPHORHALTIGE AMPHIPHILISCHE MOLEKÜLE MIT OBERFLÄCHENAKTIVEN EIGENSCHAFTEN
MOLECULES AMPHIPHILIQUES CONTENANT DU FLUOR ET DU PHOSPHORE, PRESENTANT DES PROPRIETES TENSIO-ACTIVES

(30) Priority: 22.06.1989 EP 89401777
(43) Date of publication of application: 08.04.1992
(73) Proprietor: APPLICATIONS ET TRANSFERTS DE TECHNOLOGIES AVANCEES ATTA, F-06000 Nice (FR)
(72) Inventor: RIESS, Jean, F-06950 Falicon (FR); JEANNEAUX, François, F-06100 Nice (FR); KRAFFT, Marie-Pierre, F-06100 Nice (FR); SANTAELLA, Catherine, F-06100 Nice (FR); VIERLING, Pierre, F-06950 Falicon (FR)
(74) Representative: Kedinger, Jean-Paul
(86) International application number: PCT/EP90/00991
(87) International publication number: WO 90/15807

(56) References cited:
- CH-A- 492 741
- DD-A- 222 595
- DE-A- 2 405 042
- Chemical Abstracts, vol, 110, 1989, (Columbus, Ohio, US), J Gu et al.: "Synthesis of fluoro-carbon phospholipids and the formation of their liposomes" see page 767, abstract 154749c.
- Chemical & Pharmaceutical Bulletin, vol. 35, no. 2, 1987, K Fujita et al.: "Synthesis and biological activity of fluorine-modified platelet activating factors, pages 647-653
- Chemical Abstracts, vol. 110, 1989, (Columbus, Ohio, US), see page 46, abstract 115981c.

## Description

### Background of the Invention

This invention relates to surfactants which as amphiphilic molecules have a variety of applications, in particular in the preparation of liposomes, dispersions, and emulsions such as fluorocarbon emulsions.

The achievement of an intravenously injectable oxygen-delivering system has become a major objective in biomedical research. Such a system is destined to serve as a temporary substitute for blood, but also, more generally, whenever in vivo administration of oxygen is required, as for example in cases of myocardial infarction or stroke, during cardiovascular surgery, for the preservation of isolated tissues and organs, as an adjuvant to cancer radio- and chemo- therapy and in perioperative hemodilution. Fluorocarbons presently appear to be the most promising oxygen vectors for this purpose. Fluorocarbons also have significant utility as contrast enhancement agents, such as for diagnosis by X-ray, magnetic resonance or ultrasound radiography.

The intravenous injection of neat fluorocarbons is, however, precluded by their insolubility in an aqueous medium. It is therefore necessary to prepare them in the form of emulsions, which implies the use of one or more surfactants. Although albumin has been used as a surfactant, the primary synthetic surfactants used in fluorocarbon emulsions today are polyoxyethylene polyoxypropylene block co-polymers of PLURONIC F-68 (registered trademark) type, and natural surfactants such as egg-volk lecithins.

Lecithins, however, have their drawbacks and limitations; they are sensitive, oxidizable materials; reliable sources of consistent quality are few; they are not particularly fluorophilic; and they leave little room for manipulating the emulsions' characteristics in order to adjust them to specific therapeutic applications.

Further mastery of the art of fluorocarbon emulsion technology is desirable, especially to allow the optimal adaptation of the emulsions' characteristics to each individual therapeutic application and to extend their spectrum of application. A further, ideal, objective would be the ability to modulate the biological response they trigger in the organism.

Likewise it is desirable to gain further mastery in the art of liposome technology, especially to allow the modulation of the characteristics and properties of lipid membranes and liposomes and to extend their spectrum of applications, especially for drug and contrast agent delivery.

The present invention provides various fluorine-substituted lecithin analogues and derivatives, which are useful as surfactants in fluorocarbon emulsions and in lipid membranes and in liposome manufacturing.

Certain fluorine-containing surfactants are known. For example, DE-A-2160783 discloses certain fluorocarbon phosphoric acid derivatives having a chlorine atom substituted on the carbon atom β- to the phosphate group.

Fujita et al. (JP-A-60181093, Chem. Pharm. Bull., 35:647 (1987) disclose certain fluorocarbon phosphoric acid derivatives based on glycerol in which a single fluorine-containing (R_{F}) moiety is present and the secondary alcohol function is either free (OH) or acetylated (OCOCH₃). DD-A-222595 discloses some fluorinated glycerophosphocholine derivatives but these contain only a 2,2,2-trifluorethyl group.

The article by Gu et al [Chemical distract 110:154749c (1989); HUAXUE XUEBAO or Acta Chimica Sinica, 49:913 (1988)], discloses phosphatidylcholine derivatives having two F-alkyl chains, but these contain a chlorine atom at their extremity.

Kunitake et al (Memoirs of the Faculty of Engineering, Kyushu University 46 221 (1986)) disclose fluorocarbon phosphoric acid derivatives which contain an amide linkage, as a result of being a glutamic acid diester.

DE-A-2656429 discloses certain fluorocarbon phosphorous (not phosphoric) acid derivatives including the presence of a CH=CF double bond.

Various publications also disclose one or two fluorocarbon moieties substituted onto a phosphoric acid moiety; in the case of the mono-substituted compounds both remaining groups of the phosphoric acid are independently hydroxy, alkoxy, alkylthio or alkylamino.

DE-A-3609491, DE-A-3609492 and JP-A-84204197 disclose certain dibasic fluorocarbon-substituted phosphoric acid derivatives.

JP-A-8623590 and JP-A-86123591 (Fuji) disclose certain fluorocarbon-substituted phosphoric acid derivatives having no methylene groups.

Mahmood et al (Inorg. Chem. 25 4081 (1986)) discloses molecules having central bifunctional fluorinated chains with two phosphate groups, one on each end of the chain.

Various sulphonamides containing fluorocarbon moieties and a phosphoric acid residue are known.

US-A-3976698 and US-A-3948887 (Pennwalt) disclose certain sulphur-containing fluorocarbon-substituted phosphoric acid derivatives.

None of the above documents discloses the use of the surfactants disclosed in fluorocarbon emulsions. Further, none of the prior documents discloses compounds within the scope of the present invention.

### Summary of the Invention

The invention is directed toward novel surfactants having the general formula 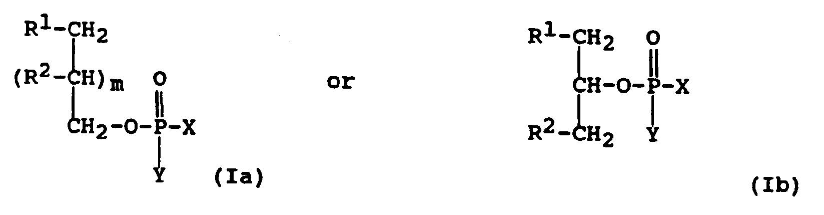
wherein R¹ and R² are fluorine containing moieties, and X and Y substituents are as defined herein.

The invention is also directed to methods of using the novel compounds described. The amphiphilic nature of the molecules combined with their biocompatibility make them useful in the preparation of emulsions and liposomal formulations which can be adapted to many biological and medical applications.

Finally. methods of preparing the compounds of Formula I are provided herein.

### Brief Description of the Drawings

The description makes reference to the accompanying drawings, in which:
Figure 1 shows the structures of preferred compounds of the invention;
Figure 2 shows a general synthetic scheme for preparing compounds of general formulae Ia and Ib and certain intermediate compounds;
Figure 3 shows an exemplary synthetic scheme for preparing certain compounds of the invention, which may be extended by analogy for the preparation of other compounds of the invention;
Figure 4 shows in more detail part of the synthetic scheme shown in Figure 3;
Figure 5 shows further exemplary synthetic schemes for preparing certain compounds of the invention, which again may be extended by analogy for the preparation of other compounds of the invention.

### Detailed Description of the Invention

According to a first aspect of the invention, there is provided a compound of the general formula: 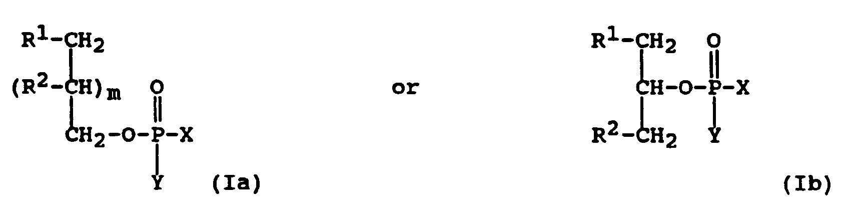
wherein:
R¹ represents:
R_{F}(CH₂)ₐ-(CH=CH)_{b}-(CH₂)_{c}-(CH=CH)_{d}-(CH₂)ₑ-A-;
R_{F}-(CH₂)_{f}-OCH₂CH(CH₂OH)CH₂-A-;
R_{F}-(CH₂)_{g}-OCH₂CH(CH₂OH)-A-,
wherein -A- represents -O-, -C(O)O-, -R⁶(R⁷)N⁺-,
(wherein each of R⁶ and R⁷ represents C₁-C₄ alkyl or hydroxyethyl), -(CH₂)ₙ- , wherein n=0 or 1, or
-C(O)N(R⁹)-(CH₂)_{q}-B, wherein q is an integer from 0 to 12, B represents -O- or -C(O)-, and R⁹ is hydrogen or R⁶,
and wherein the sum of a+c+e is from 0 to 11, the sum b+d is from 0 to 12 and each of f and g is from 1 to 12;
R_{F}-(CH₂-CH₂-O)ₕ-;
R_{F}-[CH(CH₃)CH₂O]ₕ-;
R_{F}(-CH₂-CH₂-S)ₕ-;
wherein h is from 1 to 12; and
wherein R_{F} represents a fluorine-containing moiety having one of the following structures:
(a) F(CF₂)ᵢ-, wherein i is from 2 to 12,
(b) (CF₃)₂CF(CF₂)ᵢ-, wherein j is from 0 to 8,
(c) R_{F}1[CF₂CF(CF₃)]ₖ-, wherein k is from 1 to 4, and R_{F}1 represents CF₃-, C₂F₅- or (CF₃)₂CF-,
(d) R_{F}2(RF₃)CFO(CF₂CF₂)ₗ-, wherein l is from 1 to 6 and wherein each of R_{F}2 and R_{F}3 independently represents CF₃-, C₂F₅-, n-C₃F₇- or CF₃CF₂CF(CF₃)-, or R_{F}2 and R_{F}3 taken together represent -(CF₂)₄- or -(CF₂)₅-, or
(e) one of the structures (a) to (d) in which one or more of the fluorine atoms are replaced by one or more hydrogen or bromine atoms and/or at least two chlorine atoms in a proportion such that at least 50% of the atoms bonded to the carbon skeleton of RF are fluorine atoms, and wherein R_{F} contains at least 4 fluorine atoms;

m is 0 or 1;
R² represents R¹, hydrogen or a group OR,
wherein R represents a saturated or unsaturated C₁-C₂₀ alkyl (preferably C₁-C₈ alkyl) or C₃-C₂₀ acyl (preferably C₃-C₈ acyl); and when m is 1, R¹ and R² may exchange their positions; and
each of X and Y independently represent:
hydroxyl;
-O(CH₂CH₂O)ₙR³,
wherein n is an integer from 1 to 5 and R³ represents a hydrogen atom or C₁-C₄ alkyl group;
-OCH₂CH(OH)CH₂OH;
-NR⁴R⁵ or N⁺R⁴R⁵R⁸,
wherein each of R⁴, R⁵ and R⁸ independently represents a hydrogen atom, a C₁-C₄ alkyl group, -CH₂CH₂O(CH₂CH₂O)ₛR³, wherein s represents an integer of from 1 to 5, or R⁴ and R⁵ when taken together represent -(CH₂)_{q} where q is an integer of from 2 to 5, or with the nitrogen atom R⁴ and R⁵ form a morpholino group;
-O(CH₂)ₚZ wherein Z represents a 2-aminoacetic acid group, -NR⁴R⁵ or -NR⁴R⁵R⁸ where R⁸ is as defined for R⁴ and R⁵ above, and p is an integer of from 1 to 5;
with the proviso that X and Y do not both represent hydroxyl or an ionized form derived from hydroxyl.

It is to be appreciated that at least some of the compounds of general formulae Ia and Ib can exist in ionized or non-ionized form. The exact nature of the species present will of course depend on the environment and in particular the pH.

For a better understanding of the invention, and to show how it may be put into effect, preferred embodiments of the invention, in its first and other aspects, will now be described.
Preferred compounds of general formulae Ia and Ib have, independently or (where compatible) together, one or more of the following characteristics:
- in general formula Ia, m=0;
- in general formula Ia, m=1;
- R² represents R¹;
- R¹ represents a group
   R_{F}(CH₂)ₐ-(CH=CH)_{b}-(CH₂)_{c}-(CH=CH)_{d}-(CH₂)ₑ-A-;
   - preferably b+d=0.
   - preferably -A- represents -O-, -C(O)O-, or
   - (CH₂)ₙ- (wherein n=0);
- R_{F} represents any of the previously defined structures (a) to (d), where one or more of the fluorine atoms are replaced by one or more hydrogen or bromine atoms;
- R_{F} represents F(CF₂)ᵢ-, wherein i is from 2 to 12;
- preferably R_{F} represents F(CF₂)ᵢ-, wherein i is from 4 to 8;
- each of X and Y independently represents hydroxyl, morpholino, a group OCH₂CH(OH)CH₂)OH, or a group (O(CH₂CH₂O)ₙR³, wherein n is 1 or 2 and R³ represents methyl;
- each of X and Y independently represents -0CH₂CH₂N⁺(CH₃)₃; and
- each of X and Y independently represents -O(CH₂)ₚZ where p is an integer from 1 to 5, and preferably 2, and Z represents -NR⁴R⁵ or NR⁴R⁵R⁸ where each of R⁴, R⁵ and R⁸ represents a methyl or an ethyl group;

with the proviso that X and Y do not both represent hydroxyl or an ionized form derived from hydroxyl.

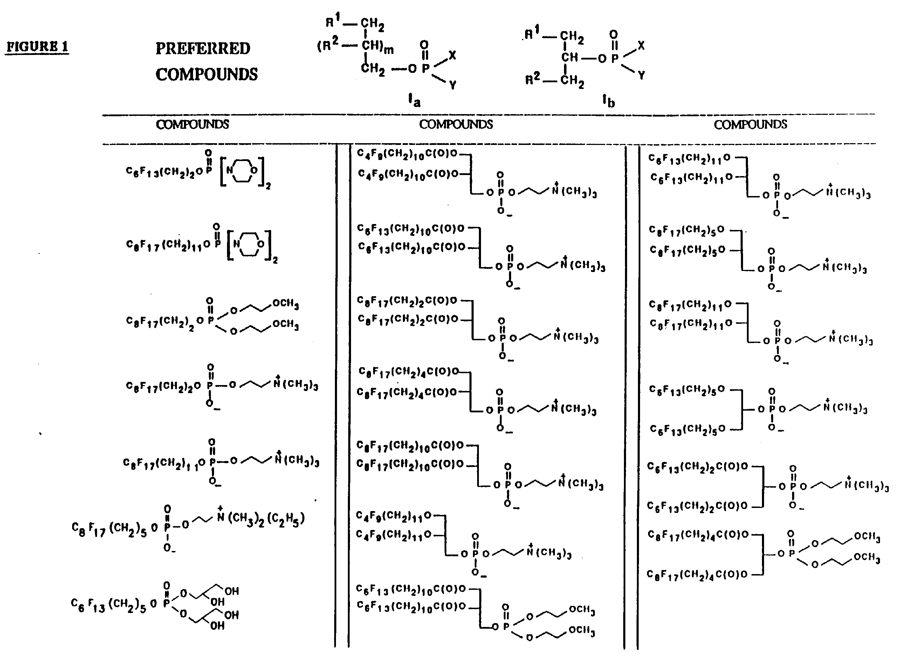

Particularly preferred compounds in accordance with the invention are shown in Figure 1.

Compounds in accordance with the first aspect may be prepared by any convenient method. Certain methods of preparing such compounds however will be preferred as a matter of practice.

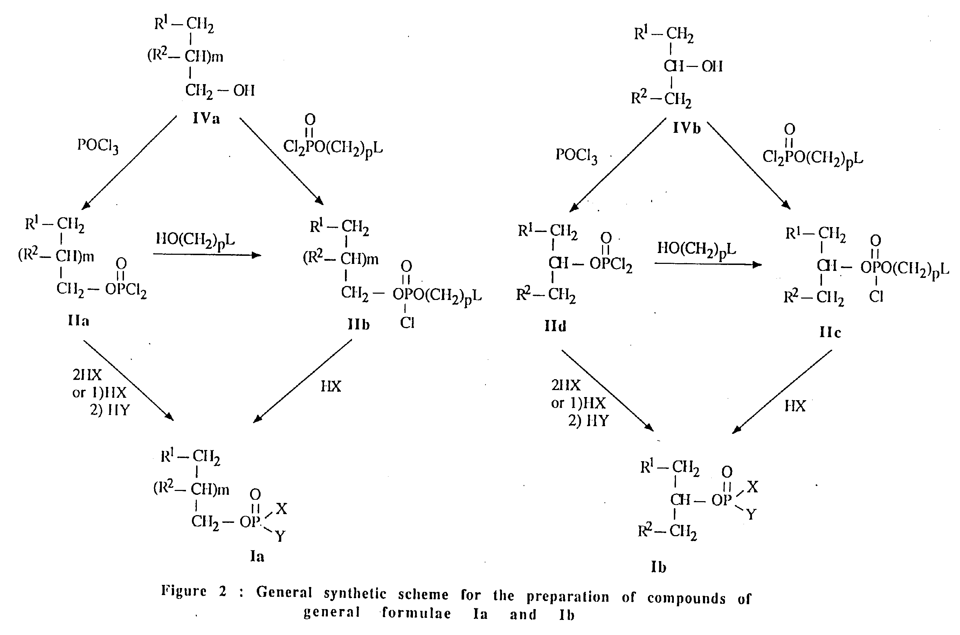

According to a second aspect of the present invention, there is provided a process for the preparation of a compound in accordance with the first aspect, the process comprising:
(a) reacting a compound of general formula IIa, IIb, IIc, or IId, as shown in Figure 2, with a compound HX to effect mono-substitution, and in the case of IIa and IIb, working up the mono-chlorinated product to a mono-hydroxylated product, and optionally allowing the product to react with the HY to effect di-substitution; or
(b) when X and/or Y represents a group -O(CH₂)ₚZ, wherein p is an integer from 1 to 5 and Z represents a group NR⁴R⁵ or N⁺R⁴R⁵R⁸, reacting a compound of general formula IIb or IIc, as shown in Figure 2, wherein L represents Z or a leaving group, and when L is Z with HX, and when L is a leaving group with HX, then with a compound HNR⁴R⁵, HN⁺R⁴R⁵R⁸, or NR⁴R⁵R⁸, to effect mono- or di-substitution and In the case of mono-substitution of a compound of general formula IIb or IIc working up the mono-chlorinated product to a mono-hydroxylated product;
(c) optionally after step (a) or (b) converting a compound of general formula Ia or Ib so formed into another compound of general formula Ia or Ib.

Compounds of general formulae IIb and IIc may be prepared from compounds of general formulae IIa and IId respectively, as shown in Figure 2, by reaction with a compound of the general formula HO(CH₂)ₚL, wherein p is defined as for general formulae Ia an Ib and L represents Z, or a leaving group, for example a halogen atom such as bromine.

Compounds of general formulae IIb and IIc may also be prepared from compounds of the formulae IVa and IVb respectively by reaction with a compound of the general formula Hal₂P(O)O(CH₂)ₚL, where p is as defined for formulae Ia and Ib, L represents Z or a leaving group as before and Hal represents a halogen atom such as chlorine, which is either available in the art or may be synthesized by methods known to those skilled in the art.

Compounds of general formulae IIa and IId can be prepared from compounds of general formulae IVa and IVb, respectively, as shown in Figure 2, by reaction with phosphorus oxychloride (POCl₃). Compounds of general formulae IVa and IVb and the other reagents used are either available in the art or may be synthesized by methods known to those skilled in the art.

Compounds of general formulae IIa, IIb, IIc, and IId are valuable intermediates in the preparation of compounds of general formulae Ia and Ib. According to a third aspect of the present invention there is provided a compound of general formula IIa or IId; according to a fourth aspect there is provided a compound of general formula IIb or IIc.

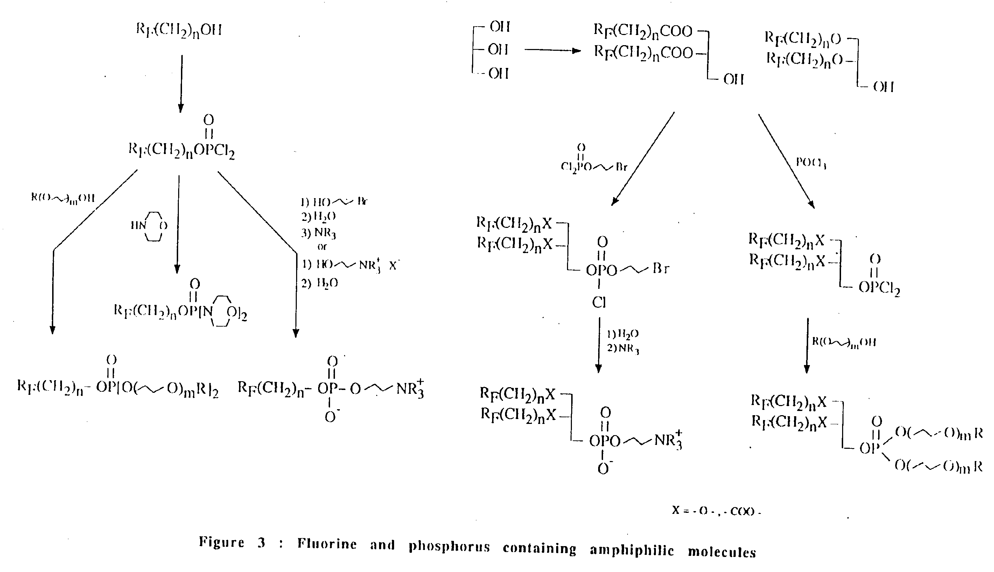

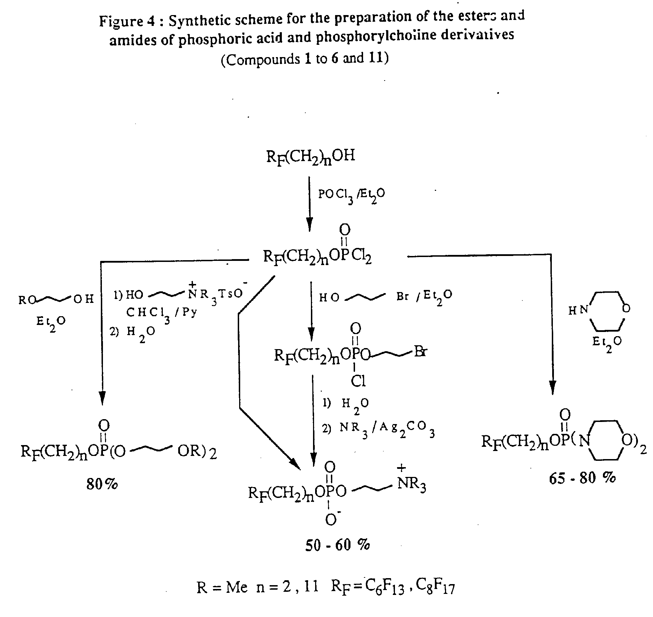

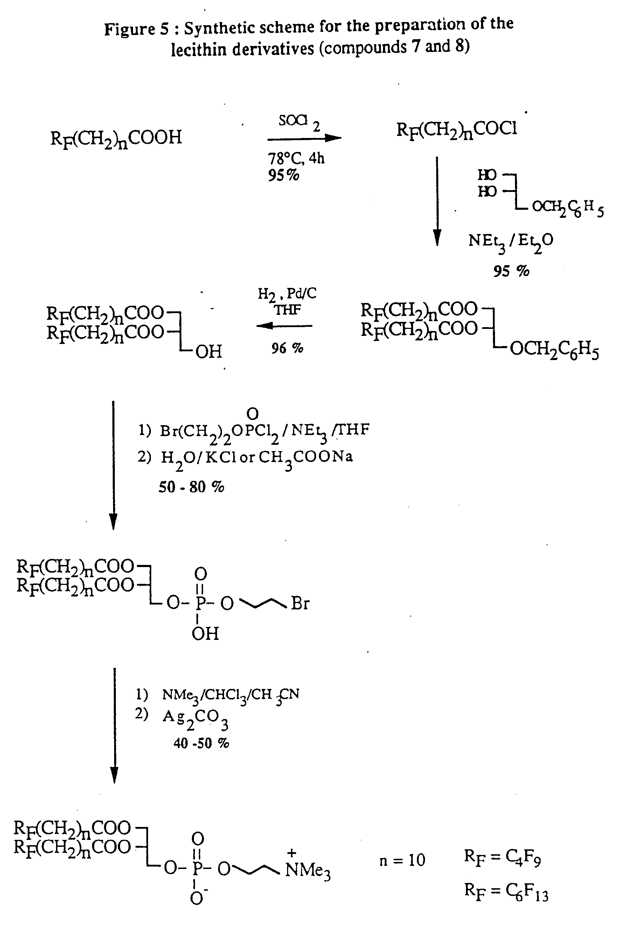

The above and other synthetic routes are illustrated in Figures 3 to 5, the procedures of which may be generalized to make other compounds of the invention.

Compounds of the invention are useful in the preparation of fluorocarbon emulsions, which in turn are useful as oxygen-carrying blood substitutes among other medical and diagnostic applications. Processes by which such emulsions can be prepared will be familiar to those skilled in the art and include the use of mechanical high pressure homogenizers such as a Gaulin homogenizer, a Microfluidizer™ (Microfluidics, Inc., Boston, Massachusetts) or even, if appropriate and economically feasible, ultrasonics. Particularly suitable preparative techniques are disclosed in EP-A-0231070 and EP-A-0307087 (both in the name of David M. Long, Jr.); compounds in accordance with the first aspect of this invention should be substituted for the surfactants disclosed in the above European patent applications (or in any other known and suitable formulation) in the same or suitably modified amounts. To the extent that the law allows, EP-A-0231070 and EP-A-0307087 are both incorporated by reference.

According to a fifth aspect of the invention, there is provided an emulsion comprising an oily phase, an aqueous phase and a surfactant in accordance with the first aspect.

Various appropriate additives may also be present, for example those disclosed in EP-A-0231070 and EP-A-0307087.

Compounds of the invention are also useful in the preparation or modification of lipid membranes and liposomes or niosomes, which in turn are useful as drug, or drug carriers, ( including in connection with oxygen carriers such as hemoglobin or modified hemoglobin or synthetic chelates), contrast agents, delivering and targeting systems, or in cosmetics. Processes by which such lipid membranes, liposomes or niosomes can be prepared will be familiar to those skilled in the art and include the use of solvent techniques, injection, or the use of ultrasonics or of a mechanical high pressure homogenizer such as a Gaulin homogenizer or a Microfluidizer™. Thus, the present invention provides a stable, non-hemolytic liposomal formulation comprising any one of the compounds in accordance with the first aspect, possibly together with a therapeutic, cosmetic or diagnostic agent, such as a drug or oxygen.
The term "emulsion" is intended to include dispersions, liposomes, niosomes, vesicles, gels, micellar solutions, and microemulsions, or similarly structured phases, and containing polar or non-polar substances, including drugs, or an oil, which may be hydrocarbonated or not, and the emulsion may contain one or more other surfactants.

The non-polar substances or oils may be highly fluorinated or perfluorinated and present at a concentration of from 10 % to 70 % volume/volume. Thus the present invention contemplates a fluorocarbon as the oily phase, in which case such compositions are useful as blood substitutes and contrast enhancement agents. In such compositions, the highly fluorinated or perfluorinated compounds, with molecular weights between about 400 and 700, may be chosen especially, but not exclusively, among at least one of the following: the bis(F-alkyl)-1,2-ethenes and more particularly the bis(F-butyl)-1,2-ethenes, the F-isopropyl-1, F-hexyl-2-ethenes and the bis(F-hexyl)-1,2 -ethenes, the perfluorodecalins, the perfluoro-methyldecalins, the perfluoro-dimethyldecalins, the perfluoromono- and dimethyladamantanes, the perfluoro-trimethylbicyclo/3,3,1/nonanes ant their homologues, ethers of formula (CF₃)CFO(CF₂CF₂)OCF(CF₃)₂, (CF₃)₂CFO(CF₂CF₂)₃OCF(CF₃)₂, (CF₃)₂CFO(CF₂CF₂)₂F, (CF₃)₂CFO(CF₂CF₂)₃F, F[CF(CF₃)CF₂O]₂CHFCF₃, (C₆F₁₃)₂O, the amines N(C₃F₇)³, N(C₄F₉)₃, the perfluoromethyl-quinolidines and perfluoroisoquinolidines, the halogen derivatives C₆F₁₃Br, C₈F₁₇Br, C₆F₁₃CBr₂CH₂Br, 1-bromoheptadecafluoro-4-isopropylcyclohexane or mixed hydrocarbon/fluorocarbon compounds with a lower molecular mass, CₙF₂ₙ₊₁CₘN₂ₘ₊₁, CₙF₂ₙ₊₁CₘH₂ₘ₋₁, in which the hydrocarbon chain contains a double bond, wherein n is an integer between 1 and 10 and m is an integer between 1 and 20 and analogues, it being understood that the compounds can be used separately or in the form of mixtures. Such compositions are more particularly used as gas carriers, and in particular for oxygen in living surroundings, for human and veterinary medical applications, in particular as blood substitutes, means to treat cerebral and cardiac ischemia in preoperative hemodilution, for the preservation of organs, tissues, embryos, semen, medium usable in cardiovascular therapy and surgery, for example as a cardioplegic, reperfusion, or coronary angioplasty, solution medium usable as adjuvant for radiotherapy or chemotherapy of cancer, or medium usable as medicinal vehicle, as contrast agents or diagnosis by X-rays, magnetic resonance or ultrasound radiography.

The compositions of the present invention may comprise 5-80% (vol/vol) of the oily phase, e.g., a non-polar compound, and 0.5-12% (vol/vol) of at least one surfactant of the first aspect, and the remainder being the solvent, e.g. water, and optionally, various additives, including inorganic salts, generally in the form of buffers, which allow adjustment of the pH and obtaining of an isotonic composition.

The surfactant comprises at least one of the fluorinated surfactants of the first aspect of the present invention, optionally in combination with conventional surfactant, the fluorinated surfactants of the invention representing, by volume, from 1% to 100% of the total volume of surfactants. The present invention is illustrated by means of the following Examples, which are not intended to be unduly limiting, since the methods set forth therein are broadly applicable to the preparation of all of the compounds disclosed.

### EXAMPLE 1: Synthesis of [2-(F-hexyl)-ethyl] dimorpholinophosphoramidate 1

20.89 g of 2-(F-hexyl)-ethanol and 18 ml of triethylamine were allowed to react in dry ether at 0°C and under argon with 8.8g of phosphorous oxychloride to give [2-(F-hexyl) ethoxyl] phosphoryl dichloride.

A solution of 12.5g of morpholine and 18 ml of triethylamine in ether was then added dropwise to the cooled reaction mixture. After treatment, the oily clear residue was distilled (Eb = 150°C/0.03 mmHg), yielding 26.72g (80%) of [2-(F-hexyl) ethyl] dimorpholinophosphoramidate 1.
F=25°C ± 1°C; C found (calculated) 33.40 (32.99); H 3.52 (3.44); N 4.93 (4.81); F 40.42(42.44); P 5.36 (5.83); MS (LID/IC/NH₃); m/e (%); M+1 583 (100); IR (ν cm⁻¹): 1250-1150 (P=O, C-F), 972 (P-N); NMR ¹H (δppm, CDCl₃): 2.56 (tt, 2H, ³J_{HH}=5.3 Hz, ³J_{HF}=18.5 Hz, R_{^{F}}CH₂), 3.17 (dt, 8H, ³J_{HH}=5.3 Hz, ³J_{PH}=2.7 Hz, NCH₂), 3.68 (t, 8H, ³J_{HH}=5.3 Hz, CH₂OCH₂), 4.32 (dt, 2H, ³J_{HH}=5.3 Hz, ³J_{PH}=7.9 Hz, CH₂OP); NMR ¹³C (δppm, CDCl₃): 31.9 (td, ¹J_{CF}=21 Hz, ³J_{CP}= 7 Hz, R_{F}CH₂), 44.5 (s, PNCH₂), 57.1 (d, ²J_{PC}=5 Hz, R_{F}CH₂CH₂), 67.1 (d, ³J_{PC}=8 Hz, CH₂OCH₂); NMR ³¹P (δppm, CDCl₃): 14.2; NMR ¹⁹F(δppm, CDCl₃): -81.3 (CF₃), -114.0 (CF₃CF₂), -122.3 (2F), -123.3 (2F), -124.0 (2F), -126.6 (CH₂CH₂).

### EXAMPLE 2: Synthesis of [2-(F-octyl) ethyl] dimorpholinophosphoramidate 2

The experimental procedure described above when applied to 16.58g of 2-(F-octyl)-ethanol, 5.48g of phosphorus oxychloride and 7.84g of morpholine afforded after treatment, chromatography and/or recrystallization from hexane, 17.04g (70%) of 2 as white crystals.
F=60°C ± 1C; C 31.73 (31.67); H 2.96 (2.93); N 3.90 (4.10); F 47.21 (47.36); P 4.23 (4.54); MS (LID/IC/NH₃); m/e (%), M + 1: 683 (100); IR (ν cm⁻¹): 1250 - 1150 (P=O, C-F), 970 (P-N): NMR ¹H (δppm, CDCl₃): 2.54 (tt, 2H, ³J_{HH}= 5.3 Hz, ³J_{HF}=18.5 Hz, R_{F}CH₂) ; 3.16 (dt, 8H, ³J_{HH}=5.3 Hz, ³J_{PH}=2.7 Hz, NCH₂); 3.66 (t, 8H, ³J_{HH}=5.3 Hz, CH₂OCH₂); 4.31 (dt, 2H, ³J_{HH}=5.3 Hz, ³J_{PH}=7.9 Hz, CH₂OP); NMR ¹³C (δppm, CDCl₃): 32.1 (td, ²J_{CF}=21.5 Hz, ³J_{CP}=7 Hz, R_{F}CH₂), 44.6 (s, NCH₂), 57.3 (m, ²J_{PC}=5 Hz, R_{F}CH₂CH₂), 67.1 (d, J_{CP}=5 Hz, CH₂OCH₂); NMR ³¹P (δppm, CDCl₃): 14.2; NMR ¹⁹F (δppm, CDCl₃): -81.3 (CF₃); -114.0 (CF₃CF₂) ; -122.4 (6F); -123.2 (2F); -124.0 (2F); -126. 6 (CF₂CH₂).

### EXAMPLE 3: Synthesis of [11-(F-hexyl) undecyl] dimorpholinophosphoramidate 3

The previous method when applied to 3.26g of 11-(F-hexyl) undecanol, 1. 02g of phosphorus oxychloride and 1.73g of morpholine, gave after chromatography 3.0g (650) of the phosphoramidate 3.
F=20°C ± 1°C; C 42.56 (42.37); H 5.24 (5.36); N 3.66 (3.95); F 34.03 (34.89); P 4.43 (4.38); MS (LID/IC/NH₃); m/e (%); M+1 683 (100); IR (ν cm⁻¹) 2929, 2954 (C-H); 1240-1150 (P=O, C-F); 972 (P-N); NMR ¹H (δppm, CDCl₃): 1.33 ("s", 18H, (CH₂)₉); 1.80 (m, 2H, R_{F}CH₂); 3.14 (dt, 8H, ³J_{HH}=5.3 Hz, ³J_{PH}=2.7 Hz, NCH₂); 3.64 (t, 8H, ³J_{HH}=5.3 Hz, CH₂OCH₂) ; 3.98 (dt, 2H, ³J_{HH}=5.3 Hz; ³J_{PH}=7.9 Hz, CH₂OP); NMR ¹³C (δppm, CDCl₃): 20.0 (t, ³J_{CF}=5 Hz, R_{F}CH₂CH₂), 25.7, 29.1, 29.2, 29.3, 29.5, 30.6 (all s, 8CH₂), 30.8 (t, ²J_{CF}=20 Hz, R_{F}CH₂); 44.7 (s, NCH₂), 65.5 (d, ²J_{CP}=4.8 Hz, CH₂OP), 67.2 (d, ³J_{CP}=6 Hz, CH₂OCH₂); NMR ³¹P (δppm, CDCl₃): 13.9; NMR ¹⁹F (δppm, CDCl₃): -81.3 (CF₃); -114.9 (CF₃CF₂), -122.3 (6F); -123.2 (2F); -124.0 (2F); -126.6 (CF₂CH₂).

### EXAMPLE 4: Synthesis of [11-(F-otcyl) undecyl) dimorpholinophosphoramidate 4

As in Example 3, the reaction between 3.5g of 11-(F-ocyyl) undecanol-1, 0.91g of phosphorus oxychloride and 1.3g of morpholine, afforded, after treatment and chromatography 3.40g (71%) of the phosphoramidate 4.
F=65°C ± 1°C; C 40.08 (40.10); H 4.83 (4.70); N 3.43 (3.46); F 38.50 (39.97); P 3.75 (3.84); IR (ν cm⁻¹): 2924, 2853 (C-H); 1258-1205 (P=O, C-F); 974 (P-N); NMR ¹H (δppm, CDCl₃): 1.32 (broad s, 18H, (CH₂)₉; 2.03 (m, 2H, R_{F}CH₂); 3.16 (dt, 8h, ³J_{HH}=5.3 Hz, ³J_{PH}=2.7 Hz, NCH₂); 3.66 (t, 8H, ³J_{HH}=5.3 Hz, CH₂OCH₂); 3.96 (dt, 2H, ³J_{HH}=5.3 Hz, ³J_{PH}=7.9 Hz, CH₂OP); NMR ¹³C (δppm, CDCl₃): 20.5 (t, ³J_{CF}=5 Hz, R_{F}CH₂CH₂), 26.0, 29.3, 30.0, 30.1, 30.2, 31.5 (all s, 8 CH₂), 31.7 (t, ²J_{CF}=20 Hz, R_{F}CH₂), 44.8 (s, NCH₂), 65.7 (d, ²J_{CP}=5 Hz, CH₂OP), 67.5 (d, ³J_{CP}=6 Hz, CH₂OCH₂); NMR ³¹P (δppm, CDCl₃): 13.9; NMR ¹⁹F (δppm, CDCl₃): -81.3 (CF₃); -114.9 (CF₃CF₂); -122.3 (6F); -123.2 (2F); -124.0 (2F); -126.6 (CF₂CH₂).

### EXAMPLE 5: Synthesis of [2-(F-octyl) ethyl] [2'-N,N,N trimethylamino ethyl] phosphate 5

2-(F-octyl)-ethanol (21.30g) and triethylamine (14.5ml) were allowed to react in dry ether at 0°C and under argon first with phosphorus oxychloride (7.04g) then with 5.74g of bromoethanol and 10ml triethylamine to give 29.02g (94%) of [2-(F-octyl) ethoxy) [2'-bromoethyl) phosphoryl chloride.

28.86g of this compound, dissolved in acetonitrile, were hydrolyzed at 0-5°C into 27.65g (98%) of [2-(F-octyl-) ethyl) [2'-bromoethyl] phosphate.

A large excess of trimethylamine was bubbled through a 50/50 chloroform/acetonitrile solution of the latter compound. The mixture, heated at 40°C for 15 hours, was then allowed to react with silver carbonate (5.91g), leading after treatment to 18.71g (71%) of 5.
F: decomposition 267°C; C(+H₂O) 28.12 (27.82): H 3.01 (2.94); N 2.15 (2.16); F 48.29 (49.92); P 4.59 (4.79); MS (LID/IC/NH₃) m/e (%): M+1 630 (2.5); IR (ν cm⁻¹): 1250-1200 (P=O, C-F); NMR ¹H (δppm, CH₃OD); 2.55 (tt, 2H, ³J_{HH}=5.3 Hz, J³_{HF}=18.5 Hz; R_{F}CH₂); 3.24 (s, 9H, NCH₃); 3.66 (m, 2H, CH₂N); 4.28 (m, 4H, CH₂O); NMR ¹³C (δppm, CD₃OD); 33.9 (dt, ²J_{CF}=20.5 Hz, ³J_{PC}=7 Hz, R_{F}CH₂); 55.3 (3 lines due to J_{CN}=4 Hz, NCH₃); 59 (m, ³J_{PC}=5 Hz, R_{F}CH₂CH₂), 61 (d, ²J_{CP}=5.4 Hz, OCH₂CH₂N); 68.1 (m, ¹J_{CN}=4 Hz, ³J_{PC}=7 Hz, CH₂N); NMR ³¹P (δppm, CD₃OD): 0.50; NMR ¹⁹F (δppm, CD₃OD); -80.7 (CF₃); -113.0 (CF₃CF₂); -121.3 (6F); 122.2 (2F); -123.1 (2F); -125.7 (CH₂CH₂).

### EXAMPLE 6: Synthesis of [11-(F-octyl) undecyl] [2'-N,N,N trimethylamino ethyl] phosphate 6

The process of Example 5 applied first to 60.70g of 11-(F-otcyl)-undecanol, 36ml of triethylamine and 15.74g of phosphorus oxychloride, then to 12.86g of bromoethanol and 20ml of triethylamine yielded 78.42g (966) of [11-(F-octyl) undecyl] 2'-bromoethyl) phosphoryl chloride. After hydrolysis into (11-(F-octyl) undecyl] [2'-bromoethyl] phosphate and reaction with trimethylamine, then with 17.70g of silver carbonate and successive recrystallizations from chloroform-methanol, 39.02g (50% global) of 6 were obtained.
F decomposition > 250°C; C (+H₂O) 37.14 (37.26; H 5.20 (4.78); N 2.07 (1.81); F 40.83 (41.78); P 4.20 (4.01); IR (ν cm⁻¹); 2924-2954 (C-H); 1236-1204 (P=O, C-F); NMR ¹H (δppm, CD₃OD); 1.34 (broad s, 18H, (CH₂)₉); 2.03 (tt, 2H, R_{F}CH₂); 3.22 (s, 9H, NCH₃), 3.65 (m, 2H, CH₂Br); 3.85 (dt, 2H, ³J_{PH}=6 Hz, (CH₂)₉CH₂OP); 4.26 (m, 2H, ³J_{PH}=4 Hz, OCH₂CH₂; NMR ¹³C (δppm, CD₃OD): 21.2 (J<2Hz, R_{F}CH₂CH₂), 26.9 30... 30.8 [(CH₂)₇], 31.8 (CH₂CH₂CH₂OP), 31.9 (t, ²J_{CF}=22 Hz, CF₂CH₂), 54.6 (three lines due to ¹J_{CN}=4 Hz, NCH₂), 60.15 (d, ²J_{CP} = 4.9 Hz, (CH₂)₇CH₂O), 66.75 (d, ²J_{CP} = 6.2 Hz, OCH₂CH₂N), 67.4 (m, CH₂N); NMR ¹⁹F (δppm, CD₃OD): -80.9 (CF₃); -114.0 (CF₃CF₂); -121-5 (6F); -122-3 (2F); -123-1 (2F); -125.9 (CF₂CH₂); NMR ³¹P (δppm, CD₃OD); 0.50.

### EXAMPLE 7: Synthesis of [5-(F-hexyl) pentyl] [2'N,N,N trimethylamino ethyl] phosphate 7

5-F-hexyl) pentanol (3.1g) and triethylamine (1.3ml) were allowed to react in dry ether at 0°C and under argon with phosphorous oxycholoride (1.42g). After evaporation of the solvent and redissolution in dry chlorofrom, a solution of choline tosylate (3-0g) in pyridine (5.2ml) was added.

After hydrolysis and treatment, 3.2gm (73% of 7 were obtained.
C 33.71 (33.64); H 4.09 (4.06); N 2.44 (2.45); F 41.20 (43.23); P 5.50 (5.42); RMN ¹H (δppm, CD₃OD, TMS): 1.32-1.80 (m, 6H, R_{F}CH₂(CH₂)₃), 1.98-2.30 (m, 2H, R_{F}CH₂-), 3.26 (s, 9H, N(CH₃)₃), 3.70 (m, 2H, -CH₂N, 3.90 (dt, ³J_{HH}=6.6Hz, ³J_{HP}=5.5Hz, 2H, R_{F}(CH₂)₄CH₂OP), 4.28 (m, 2H, OCH₂CH₂N); RMN ¹³C(δppm, CD₃OD, TMS): 21.0 (t, ³J_{CF}=4.7Hz, CF₂CH₂CH₂); 26.5 (s, R_{F}(CH₂)₂CH₂), 31.4 (d, ³J_{CP}=7.2Hz, R_{F}(CH₂)₃CH₂CH₂OP), 31.6 (t, ²J_{CF}=22.3Hz, R_{F}CH₂), 54.6 (t, ¹J_{CN}=3.7Hz, N(CH₃)₃, 60.2 (d, ²J_{CP}=4.9Hz, OCH₂CH₂N) 66.4 (d, ²J_{CP}=6.1Hz, R_{F}(CH₂)₄CH₂OP), 67.4 (m, OCH₂CH₂N); RMN ¹⁹F (νppm, CD₃OD, CFCl₃): -81.3 (3F, CF₃), -114.1 (2F, CF₂CH₂), -121.7 to 123.2 (6F, CF₃CF₂(CF₂)₃), -126.2 (2F, CF₃CF₂); RMN ³¹P (δppm, CD₃OD, H₃PO₄): 0.74(s).

### EXAMPLE 8: Synthesis of [5-(F-octyl) pentyl] [2'N,N,N trimethylamino ethyl] phosphate 8

The process of Example 7 applied first to 10.1g of 5-(F-octyl) pentanol, 3.5ml of triethylamine and 3.85g of phosphorus oxychloride, then to 8.25g of choline tosylate and 12ml of pryridine yielded after hydrolysis and treatment 9.4g (70%) of 8.
C 32.20 (32.20); H 3.78 (3.45); N 2.06 (2.09); F 44.82 (48.40); P 4.80 (4.61); RMN ¹H (δppm, CD₃OD, TMS): 1.45-1.80 (m, 6H, R_{F}CH₂(CH₂)₃); 2.05-2.37 (m, 2H, R_{F}CH₂-); 3.27 (s, 9H, N(CH₃)₃); 3.68 (m, 2H, -CH₂N); 3.94 (dt, ³J_{HH}=6.0 Hz, ³J_{HP}=6.3 Hz, 2H, R_{F}(CH₂)₄CH₂OP); 4.28 (m, 2H, OCH₂CH₂N); RMN ¹³C (δppm, CD₃OD, TMS): 20.1 (t, ³J_{CF}=3.8 Hz, CF₂CH₂CH₂); 26.4 (s, R_{F}(CH₂)₂CH₂); 31.4 (d, ³J_{CP}=7.4 Hz, R_{F}(CH₂)₃CH₂CH₂OP); 31.6 (t, ²J_{CF}=22.1 Hz, R_{F}CH₂); 54.6 (t, ¹J_{CN}=4.0 Hz, N(CH₃)₃); 60.2 (d, ²J_{CP}=4.8 Hz, OCH₂CH₂N); 66.4 (d, ²J_{CP}=6.2 Hz, R_{F}(CH₂)₄CH₂OP); 67.5 (m, OCH₂CH₂N); RMN ¹⁹F (δppm, CD₃OD, CFCl₃): -81.0 (3F, CF₃); -113.9 (2F, CF₂CH₂); -121.4 to -123.0 (10F, CF₃CF₂(CF₂)₃); -125.9 (2F, CF₃CF₂); RMN ³¹P (δppm, CD₃OD, H₃PO₄): 1.18 (s).

### EXAMPLE 9: Synthesis of [5-(F-octyl) pentyl] [2'-N, ethyl-N,N dimethylamino ethyl] phosphate 9

The process of Example 7 applied first to 5.2g of 5-(F-octyl) pentanol, 1.8ml of triethylamine and 1.96g of phosphorus oxychloride, then to 4.45g of N-ethyl-n,n-dimethyl-ethanolamine tosylate and 6.2ml of pyridine yielded after hydrolysis and treatment 4.7g (67%) of **9**.
RMN ¹H (δppm, CD₃OD, TMS): 1.40 (t, ³J_{HH}=7.2 Hz, 3H, NCH₂CH₃); 1.45-1.80 (m, 6H, R_{F}CH₂(CH₂)₃); 2.04-2.33 (m, 2H, R_{F}CH₂-); 3.16 (s, 6H, N(CH₃)₂); 3.52 (q, ³J_{HH}=7.2 Hz, 2H, NCH₂CH₃); 3.60 (m, 2H, -CH₂N); 3.90 (dt, ³J_{HH}=6.1 Hz, ³J_{HP}=6.3 Hz, 2H, R_{F}(CH₂)₄CH₂OP); 4.22 (s large, 2H, OCH₂CH₂N); RMN ¹³C (δppm, CD₃OD, TMS): 8.5 (s, NCH₂CH₃); 21.1 (t, ³J_{CF}=4.6 Hz, CF₂CH₂CH₂); 26.5 (s, R_{F}(CH₂)₂CH₂; 31.5 (d, ³J_{CP}=7.4 Hz, R_{F}(CH₂)₃CH₂CH₂OP); 31.7 (t, ²J_{CF}=22.2 Hz, R_{F}CH₂); 51.6 (s large, N(CH₃)₂); 60.1 (d, ²J_{CP}=5.1 Hz, OCH₂CH₂N); 62.3 (s large, NCH₂CH₃); 64.6 (m, OCH₂CH₂N); 66.5 (d, ²J_{CP}=5.6 Hz, R_{F}(CH₂)₄CH₂OP); RMN ¹⁹F (δppm, CD₃OD, CFCl₃): -81.0 (3F, CF₃); -113.9 (2F, CF₂CH₂); -120.3 to -123.0 (10F, CF₃CF₂(CF₂)₃); -125.9 (2F, CF₃CF₂); RMN ³¹P (δppm, CD₃OD, H₃PO₄): 1.04 (s).

### EXAMPLE 10: Synthesis of 1.2-di[(11-F-hexyl) undecanoyl] 3-[2'-(N,N,N-trimethylamino) ethyl phosphoryl] rac glycerol 10

### 1) Synthesis of 1,2-Di[(11-F-hexyl) undecanoyl]3-benzyl rac-glycerol

1-benzyl rac-glycerol (3.92g) and triethylamine were allowed to reach in Et₂O at 0°C under argon with 11-F-hexylundecanoyl chloride (24.56g). After chromatography and recrystallization, 23.89g (95%) of 1,2-di[(11-F-hexyl) undecanoyl] 3-benzyl rac-glycerol as a white solid were obtained.
C 46.00 (45.76), H 4.389 (4.51), F 42.97 (42.77); IR (ν cm⁻¹, KBr): 1742 (C=O), 1240-1100 (CF), 735, 702 (monosubstituted benzene); NMR ¹H (δppm, CDCl₃, TMS): 1.20-2.60 (m, 40H, (CH₂)₁₀), 3.6 (d, ³J_{HH}=5.3 Hz, 2H, CH₂OBz), 4.13-4.55 (m, 2H, COOCH₂CH), 4.66 (s, 2H, CH₂Ph), 5.20-5.50 (m, 1H, CH), 7.46 (s, 5H, Ph); NMR ¹³C (δppm, CDCl₃/CD₃OD, TMS): 20.21 (t, ³J_{CF}=3.7 Hz, CF₂CH₂CH₂), 24.96 and 25.03 (s, CH₂CH₂CO), 29.19, 29.30 and 29.43 (s, (CH₂)₆), 31.03 (t, ²J_{CF}=22.3 Hz, CF₂CH₂), 34.20 and 34.41 (s, CH₂CO), 62.79 and 68.46 (s, CH₂CHCH₂), 70.21 (s, CH), 73.45 (s, CH₂Ph), 127.71 and 128.51 (s, C ortho and meta), 127.86 (s C para), 137.89 (s,. CH₂-C(Ph), 173.13 and 173.41 (s, CO).

### 2) Synthesis of 1,2-di[(11-F-hexyl)-undecanoyl] rac-glycerol.

1.44g of 10% palladium on activated charcoal were added under argon to a solution of 1.2-di[(11-F-hexyl) undecanoyl] 3-benzyl rac-glycerol (20.62g) in THF. The stirred suspension was kept under hydrogen pressure (1.6 bar) until hydrogenolysis was complete. The catalyst was filtered off and the filtrate was either concentrated or used directly in the next step. The product was stored at 4°C.

IR (ν cm⁻¹, KBr); 3500 (OH), 1742 (C=O), 1232-1100 (CF); NMR ¹H(δppm, CDCl₃, TMS): 1.16-2.60 (m, 40H,(CH₂)₁₀), 3.76 (d, ³J_{HH}=6 Hz, 2H, CH₂OH), 4.16-4.63 (m, 2H, OCH₂), 5.13 (m, 1H CH).

### 3) synthesis of 1,2-di[(11-F-hexyl) undecanoyl] 3-[2'-(N,N,N-trimethylamino) ethyl] phosphoryl rac-glycerol, 10.

A solution of 1,2-di[(11-F-hexyl) undecanoyl] rac-glycerol (2.59g) in THF was added to a cooled solution of (2-bromoethyl) dichlorophosphate (0.82g) and triethylamine (1.23g) in THF. The mixture was first stirred at room temperature, then refluxed gently. After cooling at 0°C, 4.5ml of water were added, and stirring was continued. The mixture was decanted and the aqueous phase extracted with CHCl₃. After evaporation, the crude residue (3.33g) was dissolved in CHCl₃ and CH₃CN to which 1.23g of trimethylamine was added. The mixture was heated for 24h at 50°C. After cooling, Ag₂CO₃ (0.56g) was added and stirring was continued for 3 hours. Purification over silica gel and recrystallization afforded 1.08g (32%) of **10**.
NMR ¹H (δppm, CDCl₃, TMS): 1.30 (bs, 24H, (CH₂)₆), 1.60 (m, 8H, CH₂ in β from CF₂ and CO); 1.90-2.27 (m, 4H, CF₂CH₂); 2.25-2.40 (m, 4H, CH₂CO), 3.33 (s, 9H, NCH₃), 3.63 m, 2H, CH₂N); 4.00 (dd, 2H, ³J_{HH}=6.2 Hz, ³J_{HP}=6.7 Hz, CHCH₂OP); 4.18 and 4.45 (part AB of an ABX system, ²J_{AB}=12.3 Hz, ³J_{AX}=7 Hz, ³J_{BX}=3.3 Hz, 2H, CH₂CHCH₂OP); 4.27 (m, 2H, OCH₂CH₂N), 5.25 (m, 1H, CH); NMR ¹³C (δppm, CDCl₃/CD₃OD, TMS): 20.56 (t, ³J_{CF}=3.6 Hz, CF₂CH₂CH₂), 25.30 and 25.36 (s, CH₂CH₂CO), 29.53, 29.69 and 29.81 (s, (CH₂)₆), 31.29 (t, ²J_{CF}=22.2 Hz, CF₂CH₂), 34.50 and 34.65 (s, CH₂CO), 54.45 (three lines due to ¹J_{CN}=1.7 Hz, NCH₃), 59.57 (d, ²J_{CP}=4.9 Hz, POCH₂CH₂), 63.16 (s, OCH₂CH), 64.11 (d, ²J_{CP}=5.2 Hz, CHCH₂OP), 6.95 (m, CH₂NH), 70.96 (d, ³J_{CP}=8.2 Hz, CH), 174.02 and 174.39 (s, CO); NMR ³¹P (δppm, CDCl₃/CD₃OD, H₃PO₄): -0.68; NMR ¹⁹F (δppm, CDCl₃/CD₃OD, CFCl₃): -81.53 (CF₃), -115.12 (CF₃CF₂), -122.65, -123.66 and -124.16 ((CF₂)₃), -126.83 (CF₂CH₂).

### EXAMPLE 11: Synthesis of 1,2-di[(11-F-butyl) undecanoyl] 3-[2'-(N,N,N-trimethylamino) ethyl phosphoryl] rac-glycerol 11

The procedure described in Example 10 when applied to 1-benzyl rac-glycerol (5.3g), (11-F-butyl) undecanoyl chloride (50.3g) and triethylamine (19ml) afforded 22.1g (80%) of 1,2-di [(11-F-butyl) undecanoyl] 3-benzyl rac-glycerol. Hydrogenolysis, then reaction with (2-bromoethyl) dischlorophosphate (6.03g) and triethylamine (11.04g), followed by hydrolysis, and finally, reaction with trimethylamine (19g) led to 6.60g (30%) of **11**.
C 44.36 (44.32), H 5.75 (5.64), F 32.66 (33.21), N 1.35 (1.36) P 3.14 (3.01); RMN ¹H (δppm, CDCl₃/CD₃OD, TMS): 1.30 (bs, 24H (CH₂)₆), 1.60 (m, 8H, CH₂ in β from CF₂ and CO), 1.93-2.27 (m, 4H, CF₂CH₂), 2.30 and 2.45 (two t, 4H, CH₂CO), 3.25 (s, 9H, NCH₃), 3.6-3.7 (m, 2H, CH₂N), 4.0 (dd, 2H, ³J_{HH}=6.2 Hz, ³J_{HP}=6.7 Hz, CH₂CH₂OP), 4.18 and 4.45 (part AB of an ABX system, ²J_{AB}=12.3 Hz, ³J_{AX}=7 Hz, ³J_{BX}=3.3 Hz, 2H, CH₂CHCH₂OP), 4.3-4.33 (m, 2H, OCH₂CH₂N), 5.20 (m, 1H, CH); NMR ¹³C (δppm, CDCl₃/CD₃OD, TMS): 20.44 (t, ³J_{CF}=3.6 Hz, CF₂CH₂CH₂), 25.2 (s, CH₂CH₂CO), 29.42, 29.57 and 29.70 ((CH₂)₆), 31.08 (t, ²J_{CF}=22.3 Hz, CF₂CH₂), 34.35 and 34.51 (s, CH₂CO), 54.27 (three lines due to ¹J_{CN}=1.7 Hz, NCH₃), 59.53 (d, ²J_{CP}=4.8 Hz, POCH₂), 63.03 (s, OCH₂CH), 64.02 (d, ²J_{CP}=5 Hz, CHCH₂OP), 66.82 (m, CH₂N), 70.91 (d, ³J_{CP}=8.1 Hz, CH), 173.89 and 174.24 (s, CO); NMR ³¹P (δppm, CDCl₃/CD₃OD, H₃PO₄): -0.13 (s).

### EXAMPLE 12: Synthesis of 1,2-di [(11-F-hexyl) pentanoyl] 3-[2'-(N,N,N-trimethylamino) ethyl phosphoryl] rac-glycerol 12

The procedure described in Example 10 when applied to 1-benzyl rac-glycerol (8.3g), (11-F-hexyl) pentanoyl chloride (42g) and triethylamine (13.5ml) afforded 38.5g of 1,2-di [(11-F-hexyl) pentanoyl] 3-benzyl rac-glycerol. Hydrogenogenolysis, then reaction with (2-bromoethyl) dichlorophosphate (10.73g) and triethylamine (19.73g), followed by hydrolysis, and finally, reaction with trimethylamine (31.6g) led to 10.5g (25%) of **12**.
RMN ¹H (δppm, CDCl₃/CD₃OD, TMS): 1.73 (m, 8H, CH₂ in β from CF₂ and CO), 2.01-2.29 (m, 4H, CF₂CH₂), 2.31 and 2.63 (two t, 4H, CH₂CO), 3.30 (s, 9H, NCH₃), 3.6-3.7 (m, 2H:, CH₂N), 4.0 (dd, 2H, ³J_{HH}=6.2 Hz, ³J_{HP}=6.7 Hz, CH₂CH₂OP), 4.19 and 4.63 (part AB of an ABX system, ²J_{AB}=12.3 Hz, ³J_{AX}=7 Hz, ³J_{BX}=3.3 Hz, 2H, CH₂CHCH₂OP), 4.3-4.33 (m, 2H, OCH₂CH₂N), 5.03 (m, 1H, CH); NMR ¹³C (δppm, CDCl₃/CD₃OD, TMS):19.51 (t, ³J_{CF}=3.6 Hz, CF₂CH₂CH₂, 23.98 and 24.00 (s, CH₂CH₂CO), 30.78 (t, ²J_{CF}=22.4 Hz, CF₂CH₂), 33.32 and 33.49 (s, CH₂CO), 54.05 (three lines due to ¹J_{CN}=3.7 Hz, NCH₃), 58.78 (d, ²J_{CP}=4.8 Hz, POCH₂), 62.73 (s, OCH₂CH), 63038 (d, ²J_{CP}=5 Hz, CHCH₂OP), 66.41 (m, CH₂N), 70.43 (d, ³J_{CP}=8.1 Hz, CH), 172.56 and 174.89 (s, CO); NMR ³¹P (δppm, CDCl₃/CD₃OD, H₃PO₄):0.57 (s), ¹⁹F (δppm, CDCl₃, CD₃OD, CFCl₃): -81.5 (CF₃), -115.2 (CF₃-CF₂); -122.6, -123.6, 124.2 (CF₂)3, -128.63 (CF₃-CF₂).

### EXAMPLE 13: Synthesis of 1,2-di [(11-F-butyl) undecyl] 3-[2'-(N,N,N-trimethylamino) ethyl phosphoryl] rac-glycerol, 13

### 1) Synthesis of 1,2-di [(11-F-butyl) undecyl) benzyl-3 rac-glycerol.

6g of (11-F-butyl) undecyl tosylate in ether were allowed to react with 1g of benzyl-1 rac-glycerol under phase transfer conditions (KOH, 10N/6g of (nBu)₄N⁺ HSO₄⁻). 3.2g (63%) of the title compound were obtained after 10 days of reaction and chromatography of the organic phase.
NMR ¹H (δppm, CCl₄): 1.02-2.41 (m, 40H, (CH₂)₁₀); 3.40 (m, 9H, OCH₂ and CH); 4.47 (s, 2H, CH₂Ph); 7.26 (s, 5H, Ph).

### 2) Synthesis of 1,2-di [(11-F-butyl) undecyl) 3-[2'-(N,N,N-trimethylamino) ethyl phosphoryl) rac-glycerol, 13.

The process described in Example 10 when applied to 6.7g of 1,2-di [(11-F-butyl) undecyl]benzyl-3 rac-glycerol led, after hydrogenolysis, reaction with 1.9g of (2'-bromoethyl) dichlorophosphate and 2ml of triethylamine, then hydrolysis and finally reaction with trimethylamine (7g), to 4g (56%) of 13.
NMR ¹H (δppm, CDCl₃/CD₃OD): 1.05-1.65 (m, 36H, (CH₂)₉); 1.80-2.1 (m, 4H, CF₂CH₂); 3.17 (s, 9H, NCH₃); 3.35 (t, 2H, ³J_{HH}=6.5 Hz, CH₂N); 3.40-3.63 (m, 8H, OCH₂); 3.79 (t, 2H, ³J_{HH}=6.5 Hz); 4.10-4.30 (m, 1H, CH); NMR ¹³C (δppm, CDCl₃/CD₃OD): 20.3 (t, ³J_{FC}=3.5 Hz, CF₂CH₂CH₂); 26.3 to 30.3 (nine singles for the two (CH₂)₈ chains); 31.0 (t, ²J_{FC}=22 Hz, CF₂CH₂; 54.5 (s, NCH₃); 59.4 (d, ²J_{PC}=5 Hz, CH₂OP); 65.34 (d, ²J_{PC}=5 Hz, POCH₂CH); 66.65 (d, ³J_{PC}=6.5 Hz, CH₂N); 70.83, 72.01 (s, CH₂CH₂O); 70.9 (s, CH₂OCH₂CH); 78.3 (d, ³J_{PC}=8 Hz, CH); NMR ³¹P (δppm, CDCl₃/CD₃OD): -0.07; NMR ¹⁹F (δppm, CDCl₃/CD₃OD): -81 (CF₃), -114.0 (CF₃CF₂); -124.4 (CF₂); -126.0 (CF₂CH₂).

### EXAMPLE 14: Synthesis of [1',2'-di [(11-F-hexyl) undecanoyl] rac-glyceryl] [di (2'-methoxy-ethyl)] phosphate 14

1,2-di [(11-F-hexyl) undecanoyl] 3-benzyl rac-glycerol (1.99g) in either was added dropwise at 0°C to a solution of phosphorus oxychloride (0.31g) and triethylamine (0.66g) in ether. After stirring at room temperature, 2-methoxylethanol (0.35g) in ether was added and the mixture was refluxed. Triethylammonium chloride was filtered off, the solvent removed and 15ml of a mixture of acetonitrile and acetone was added. The soluble fraction was concentrated and purified over silica gel yielding 1g (42%) of **14**.
IR (ν cm⁻¹, KBr); 1744 (C=O), 1240-1100 (CF), NMR ¹H (δppm, CDCl₃, TMS): 1.10-1.86 (broad s, 32H, (CH₂)₈); 1.87-2.06 (m, 4H, CF₂CH₂); 2.20-2.53 (m, 4H, CH₂CO); 3.46 (s, 6H, OCH₃); 3.66 (m, 4H, CH₂OMe); 4.10-4.66 (m, 8H, OCH₂); 5.16-5.50 (m, 1H, CH).

### EXAMPLE 15: Synthesis of [2-(F-octyl) ethyl] [di-(2'-methoxyethyl)] phosphate 15

The procedure described for the preparation of 145, when applied to 30.8g of 2-F-octylethanol, 18ml of pyridine, 10.2g of phosphorus oxychloride and to 11.2g of 2-methoxyethanol led to 26.5 (60%) of 15.
IR (ν cm⁻¹): 1242 (PO); 1207-1140 (CF); 979 (P-O), NMR ¹H (δppm, CDCl₃, TMS): 2.61 (m, 2H, CF₂CH₂); 3.43 (s, 6H, OCH₃); 3.66 (m, 4H, CH₃OCH₂); 4.32 (m, 6H, CH₂OP).

### EXAMPLE 16: Synthesis of [5-(F-hexyl) pentyl][diglycerol] phosphate 16

5-(F-hexyl) pentanol (5.0g) and triethylamine (2.2ml) were allowed to react in dry ether at 0°C and under argon first with phosphorus oxychloride (2.4g) then with 10g of isopropyliden glycerol and 8.2ml triethylamine to give 5.3g (60%) of [5-(F-hexyl) pentyl][diisopropyliden glycerol] phosphate.

After hydrolysis in CF₃CO₂H/H₂O 9/1 and treatment, 4.0g (85%) of 16 were obtained.

### SURFACE ACTIVITY

The strong surface activity of the compounds encompassed by this invention is illustrated in particular by the strong lowering of the surface tensions (γₛ) they cause when added to water, as shown by the examples of surface tensions (measured at 20°C and expressed in milliNewton.meter⁻¹) and calculated spreading coefficients collected in the table below:

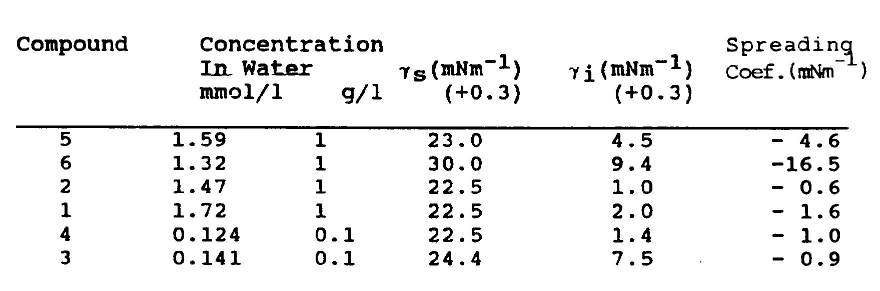

More specifically, the action of these compounds at the Interface between water and fluorocarbons is demonstrated by the very sharp diminution of the interfacial tension (γᵢ) between water and perfluorodecalin (56 mNm⁻¹ in the absence of surfactant) and the increase of the spreading coefficient (-56 mNm⁻¹ in the absence of surfactant) as illustrated by the examples collected in the same table.

### BIOCOMPATIBILITY

The biocompatibility of compounds belonging to the present invention is illustrated, in particular, by the fact that aqueous solutions or dispersions in 9% of NaCl of these compounds do not perturb the growth and multiplication of lymphoblastoid cell cultures of the Namalva strain with respect to a control of a NaCl 9% solution (100% of growth and viability).
Examples are given in the following table:

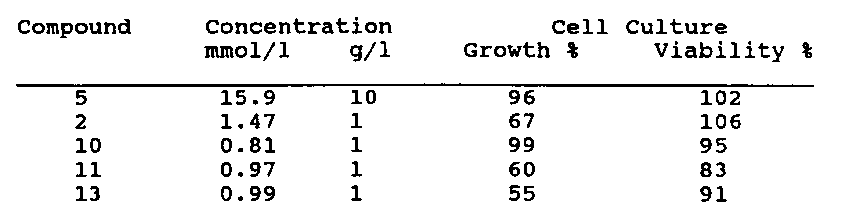

Likewise the biocompatibility of compounds belonging to the invention is illustrated by the fact that aqueous solutions or dispersions in 9% of NaCl of these compounds at the concentrations given in the following table do not cause the hemolysis of human red blood cells.

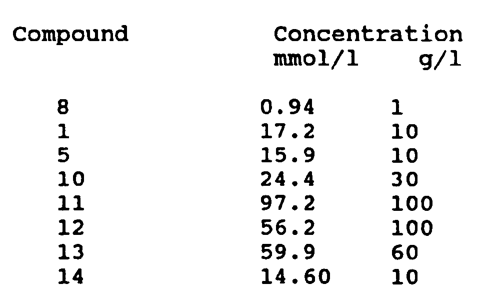

In the same way, the biocompatibility of such compounds is illustrated by the fact that the injection of 500µl of a solution or a dispersion in NaCl 9% of hereafter compounds in concentration given below, into the tail vein of 10 mice of 20-25g caused no deaths, and did not perturb the normal growth of the animals, which was observed for 35 days.

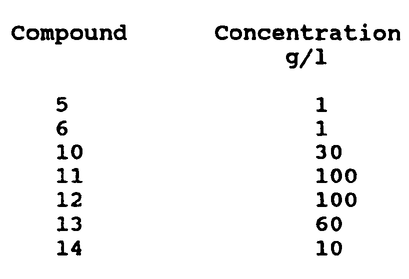

### PREPARATION OF LIPOSOMES

1) Lipid 12 dissolved in chloroform was placed in a round bottom flask and the solvent evaporated by rotation under argon to produce a uniform film of dry lipid. Residual traces of chloroform were removed under vacuum (10⁻³ mmHg, 3h). Dried lipid is suspended in HEPES buffer (10⁻² M, pH 7), vortexed for 5 mn., then probe sonicated (dial 7 on a Branson B30 sonifier, Power 4, Pulse 50, 3 mn.) 15°C above phase transition temperature, to produce a clear bluish dispersion. Final concentration of 12 is 3% (w/v). Average size measurements were realized by light scattering on a Coulter Model N4SD, sub-Micron Particle Analyzer : 0,12µm.
2) Same dispersion procedure applied to powder lipid 12 produced a clear dispersion with an average particle size 0,12µm.

Liposomes were observed by electronic microscopy after freeze-etching as unilamellar and multilamellar vesicles. The appearance of liposomes showed no differences or structural distortions after sterilization (8 mn.-121°C - 15 lb/sq.in.). Sterilized dispersions stored at 25°C showed enhanced stability as time for appearance of a precipitate monitored by visual inspection was higher than 5 months, while hydrocarbonated phosphatidylcholine dispersion's stability is known to be lower than one month.

### PREPARATION OF EMULSIONS

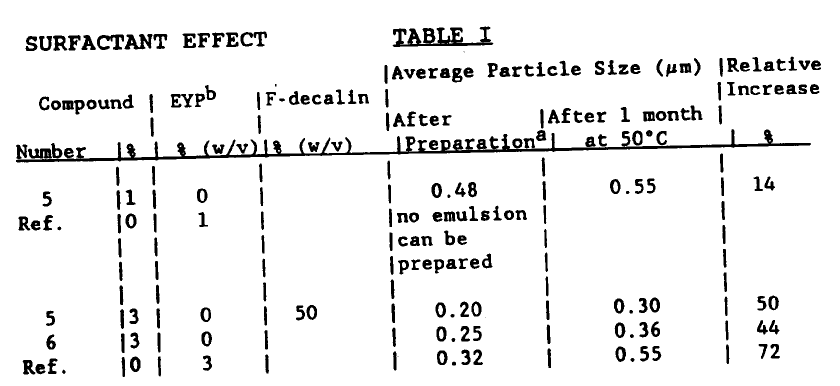

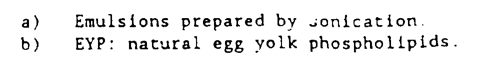

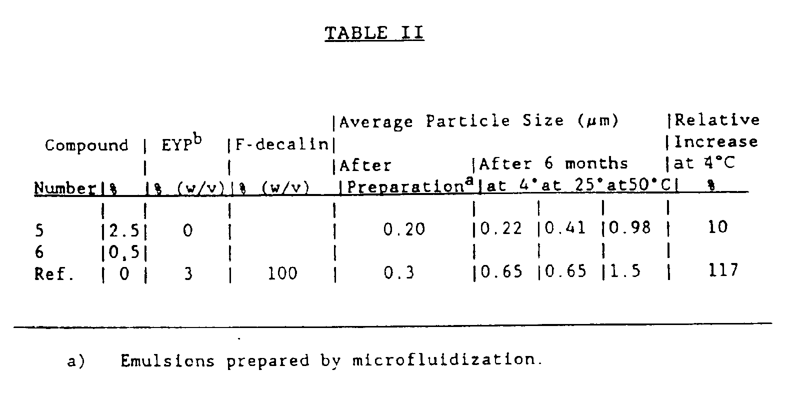

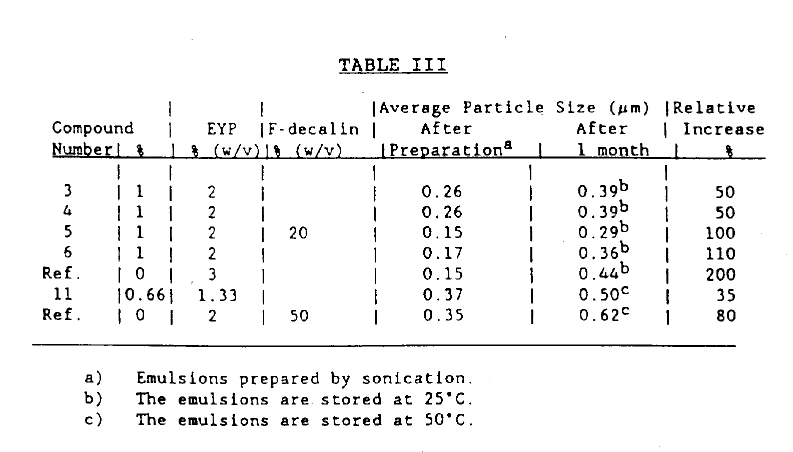

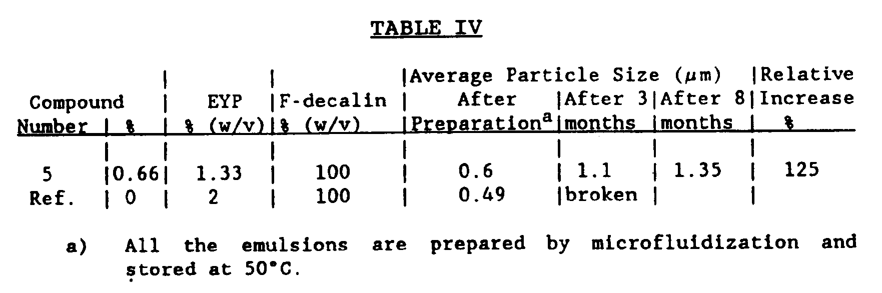

The new perfluoroalkylated surfactants were solubilized or dispersed into water. Then the fluorocarbon was added under agitation. Any emulsification method like sonication can be used but mechanical procedures such as microfluidization or high pressure homogenization are preferred. The emulsion obtained can be used as an O₂ carrier. The significant stabilization effect which can be obtained by incorporating the new F-alkyl surfactants is illustrated for various emulsion formulations (see Tables above). The results show that both the average particle sizes, measured immediately after preparation, and stability (evaluated by the relative increase of the average particle sizes, for 1 to 6 months storage at 4, 25 and 50°C) are always higher for the reference emulsions prepared with the same amount of natural EYP than for the F-alkyl-based one.

Additional stable perfluorodecalin (50 % w/v) emulsions based on the perfluoroalkylated phosphatidyl cholines 11 or 12 (2 or 3 % w/v) as the sole surfactant have been prepared by sonication. It is noteworthy that the increase in average particle size was found to be smaller for the emulsions based on the F-alkylated surfactants (10 % of increase), than for the reference emulsions.

These experiments led to several important observations: simply the fact that it is possible to prepare 50 % w/v F-decalin emulsions with F-alkylated amphiphiles as the sole surfactants, and that these emulsions are stable is, by itself, remarkable (see Table I). It is also remarkable that it proved possible to prepare such 50 % F-decalin emulsions, which remain stable at 50°C for at least one month, with only 1 % of 5. In comparison, when the same formulation is used, but with EYP instead of 5, phase separation is observed immediately (see Table I).

Another striking observation concerns the fact that at 4° C there is no detectable change in particle size in the fluorinated surfactant 5 and 6-containing highly concentrated (100 % w/v) F-decalin emulsion over a 6-month period of time (see Table II).

## Claims

1. a compound of the general formula: 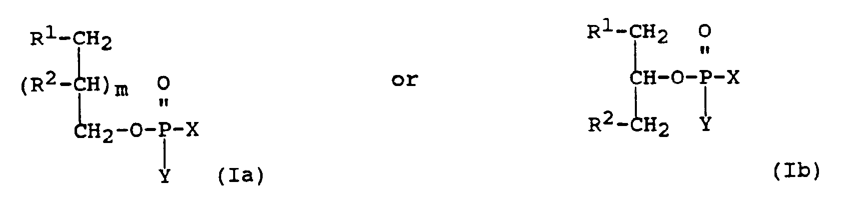 wherein:
R¹ represents:
R_{F}(CH₂)ₐ-(CH=CH)_{b}-(CH₂)_{c}-(CH=CH)_{d}-(CH₂)ₑ-A-;
R_{F}-(CH₂)_{f}-OCH₂CH(CH₂OH)CH₂-A-;
R_{F}-(CH₂)_{g}-OCH₂CH(CH₂OH)-A-;
wherein -A- represents -O-, -C(O)O-, -R⁶(R⁷)N⁺-, (wherein each of R⁶ and R⁷ represents C₁-C₄ alkyl or hydroxyethyl), -(CH₂)ₙ-, wherein n=0 or 1, or -C(O)N(R⁹)-(CH₂)_{q}-B, wherein q is an integer from 0 to 12, B represents -O- or -C(O)-, and R⁹ is hydrogen or R⁶,
and wherein the sum of a+c+e is from 0 to 11,
the sum b+d is from 0 to 12 and each of f and g is from 1 to 12;
R_{F}-(CH₂-CH₂-O)ₕ-;
R_{F}-(CH(CH₃)CH₂O)ₕ-;
R_{F}(-CH₂-CH₂-S)ₕ-,
wherein h is from 1 to 12; and
wherein R_{F} represents a fluorine-containing moiety having one of the following structures:
(a) F(CF₂)ᵢ-, wherein i is from 2 to 12,
(b) (CF₃)₂CF(CF₂)ⱼ-, wherein j is from 0 to 8,
(c) R_{F}1(CF₂CF(CF₃))ₖ-, wherein k is from 1 to 4, and R_{F}1 represents CF₃-, C₂F₅- or (CF₃)₂CF-,
(d) R_{F}2(R_{F}3)CFO(CF₂CF₂)ₗ-, wherein l is from 1 to 6 and wherein each of R_{F}2 and R_{F}3 independently represents CF₃-, C₂F₅-, n-C₃F₇- or CF₃CF₂CF(CF₃)- or R_{F}2 and R_{F}3 taken together represent -(CF₂)₄- or -(CF₂)₅-, or
(e) one of the structures (a) to (d) in which one or more of the fluorine atoms are replaced by one or more hydrogen or bromine atoms and/or at least two chlorine atoms in a proportion such that at least 50% of the atoms bonded to the carbon skeleton of R_{F} are fluorine atoms, and wherein R_{F} contains at least 4 fluorine atoms,
m is 0 or 1;
R² represents R¹, hydrogen or a group OR,
wherein R represents a saturated or unsaturated C₁-C₂₀ alkyl or C₃-C₂₀ acyl; and
when m is 1, R¹ and R² may exchange their positions; and
each of X and Y independently represent:
hydroxyl;
-OCH₂CH(OH)CH₂OH;
-O(CH₂CH₂O)ₙR³,
wherein n is an integer from 1 to 5; and
R³ represents a hydrogen atom or C₁-C₄ alkyl group;
-NR⁴R⁵ or N⁺R⁴R⁵R⁸,
wherein each of R⁴, R⁵ and R⁸ independently represents a hydrogen atom; a C₁-C₄ alkyl group;
-CH₂CH₂O(CH₂CH₂O)ₛR³, wherein s represents an integer of from 1 to 5, or R⁴ and R⁵ when taken together represent -(CH₂)_{q} wherein q is an integer of from 2 to 5, or when with the nitrogen atom R⁴ and R⁵ form a morpholino group;
-O(CH₂)ₚZ wherein Z represents a 2-aminoacetic acid group, -NR⁴R⁵ or -N⁺R⁴R⁵R⁸ where R⁸ is as defined for R⁴ and R⁵ above, and p is an integer of from 1 to 5;
with the proviso that X and Y do not both represent hydroxyl or an ionized form derived from hydroxyl.

2. The compound of Claim 1, wherein R_{F} is F(CF₂)ᵢ and m is 0.

3. The compound of Claim 1, wherein R_{F} is F(CF₂)ᵢ and m is 1.

4. The compound of Claim 3, wherein R² is the same as R¹.

5. The compound of any one of Claims 1 to 4, wherein R¹ is R_{F}(CH₂)ₐ-(CH=CH)_{b}-(CH₂)_{c}-(CH=CH)_{d}-(CH₂)ₑ-A-.

6. The compound of Claim 5, wherein b+d=0.

7. The compound of Claim 5, wherein A represents -O-, -C(O)O-, or -(CH₂)ₙ-, wherein n=0 or 1.

8. The compound of any one of Claims 1 to 4, wherein R_{F} is F(CF₂)ᵢ-, and wherein i is from 2 to 12.

9. The compound of Claim 8, wherein i is from 4 to 8.

10. The compound of any one of Claims 1 to 4, wherein each of X and Y independently represents hydroxyl, morpholino, or a group -O(CH₂CH₂O)ₙR³, wherein n is 1 or 2 and R³ represents a methyl group, with the proviso that X and Y do not both represent hydroxyl or an ionized form derived from hydroxyl.

11. The compound of any one of Claims 1 to 4, wherein each of X and Y independently represents -OCH₂CH₂N⁺(CH₃)₃.

12. A method for the preparation of a compound having the structure set forth in Claim 1, comprising the steps of:
(a) reacting a compound of general formula 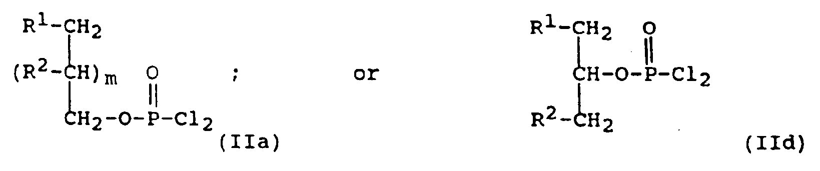 with a compound HX to effect mono-substitution of one of the Cl atoms, and converting the mono-chlorinated product to a mono-hydroxylated product,
wherein X represents:
hydroxyl;
-OCH₂CH(OH)CH₂OH;
-O(CH₂CH₂O)ₙR³,
wherein n is an integer from 1 to 5, and
R³ represents a hydrogen atom or C₁-C₄ alkyl group;
-NR⁴R⁵ or N⁺R⁴R⁵R8,
wherein each of R⁴, R⁵ and R⁸ independently represent a hydrogen atom, a C₁-C₄ alkyl group,
-CH₂CH₂O(CH₂CH₂O)ₛR³, wherein s represents an integer of from 1 to 5, or R⁴ and R⁵ when taken together represent -(CH₂)_{q} where q is an integer of from 2 to 5, or with the nitrogen atom R⁴ and R⁵ form a morpholino group;
-O(CH₂)ₚZ
wherein Z represents either a leaving group, a 2-aminoacetic acid group, -NR⁴R⁵ or -N⁺R⁴R⁵R⁸ wherein R⁸ is as defined for R⁴ and R⁵ above, and p is an integer of from 1 to 5.

13. The method of Claim 12, further comprising the step of allowing the product to react with HY to effect disubstitution, wherein Y independently represents the groups as defined for X above,
with the proviso that X and Y do not both represent hydroxyl.

14. The method of Claim 12 or 13 wherein X and/or Y represents a group -O(CH₂)ₚZ, wherein p is an integer from 1 to 5 and Z represents a leaving group, L, comprising the additional step of reacting said mono- or di-substituted product with a compound HNR⁴R⁵ or NR⁴R⁵R⁸.

15. The method of Claim 12 or 13, comprising the additional step of replacing said X or Y group of the product with a different X or Y group.

16. A stable, non-hemolytic liposomal formulation comprising any one of the compounds of Claims 1 to 4

17. A liposomal formulation comprising a compound of any one of Claims 1 to 4, together with a therapeutic, cosmetic, or diagnostic agent.

18. The formulation of Claim 17, wherein said agent is a drug or oxygen.

19. An emulsion comprising an oily phase, an aqueous phase and a surfactant, wherein said surfactant is a compound according to any one of Claims 1 to 4.

20. The emulsion of Claim 19, wherein the oily phase comprises a fluorocarbon.

21. The emulsion of Claim 20, wherein the fluorocarbon is highly fluorinated or perfluorinated, and is present in said emulsion at a concentration of from 10% to 70% volume/volume.

22. The emulsion of Claim 21, wherein the highly fluorinated or perfluorinated compound has a molecular mass of from 400 to 700 and is a bis(F-alkyl)-1,2-ethene, perfluorodecalin, perfluoro-methyldecalin, perfluoro-dimethyldecalin, perfluoromono- or dimethyladamantane, perfluoro-trimethylbicyclo-/3,3,1/nonane or homologue thereof, an ether having the formula (CF₃)CFO(CF₂CF₂)OCF(CF₃)₂, (CF₃)₂CFO(CF₂CF₂)₃OCF(CF₃)₂, (CF₃)₂CFO(CF₂CF₂)₂F, (CF₃)₂CFO(CF₂CF₂)₃F, F(CF(CF₃)CF₂O)₂ CHFCF₃, or (C₆F₁₃)₂₀, an amine which is N(C₃F₇)₃, N(C₄F₉)₃, a perfluoromethyl-quinolidine or perfluoroisoquinolidine, or a halogen derivative C₆F₁₃Br, C₈F₁₇Br, C₆F₁₃CBr₂CH₂Br, 1-bromoheptadecafluoro-4-isopropylcyclohexane or mixed hydrocarbon/fluorocarbon compounds with a lower molecular mass, CₙF₂ₙ₊₁CₘN₂ₘ₊₁, CₙF₂ₙ₊₁CₘH₂ₘ₋₁, in which the hydrocarbon chain contains a double bond, wherein n is an integer between 1 and 10 and m is an integer between 1 and 20 or analogues or mixtures thereof.

23. The emulsion of Claim 21, wherein said bis(F-alkyl)-1,2-ethene is a bis(F-butyl)-1,2-ethene, an F-isopropyl-1, an F-hexyl-2-ethene or a bis(F-hexyl)-1,2-ethene.

24. A compound having the general structure 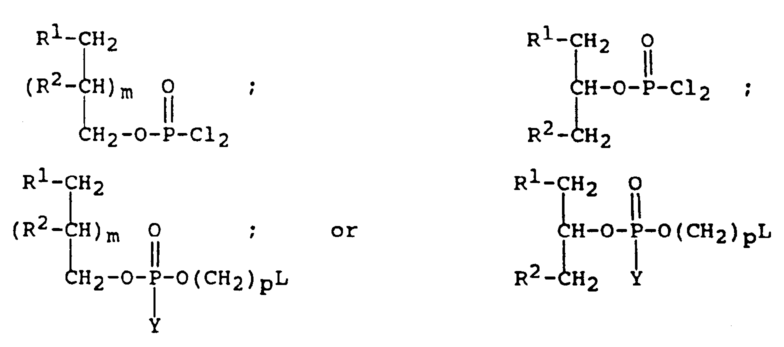 wherein R¹, R², Y, m and p have the same meaning as in claim 1 ; and L is either Z having the same meaning as in claim 1 or a leaving group.

## Patentansprüche

1. Verbindung der allgemeinen Formel: 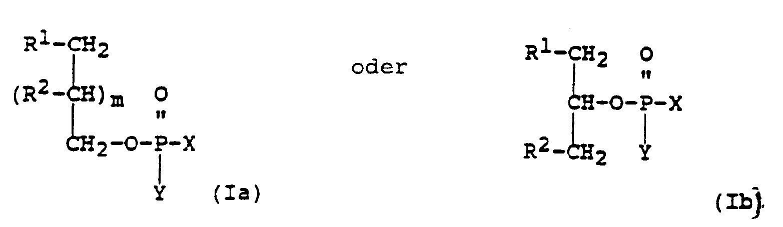 worin:
R¹ bedeutet:
R_{F}(CH₂)ₐ-(CH=CH)_{b}-(CH₂)_{c}-(CH=CH)_{d}-(CH₂)ₑ-A-;
R_{F}-(CH₂)_{f}-OCH₂CH(CH₂OH)CH₂-A-;
R_{F}-(CH₂)_{g}-OCH₂CH(CH₂OH)-A-;
worin -A- -O-, -C(O)O-, -R⁶(R⁷)N⁺-, (worin R⁶ und R⁷ jeweils C₁-C₄-Alkyl oder Hydroxyethyl bedeutet), -(CH₂)ₙ-, worin n = 0 oder 1, oder -C(O)N(R⁹)-(CH₂)_{q}-B, worin q eine ganze Zahl von 0 bis 12 ist, B -O- oder -C(O)-, bedeutet, und R⁹ Wasserstoff oder R⁶ ist, bedeutet,
und worin die Summe von a+c+e 0 bis 11 ist,
die Summe b+d 0 bis 12 ist und f und g jeweils 1 bis 12 ist;
R_{F}-(CH₂-CH₂-O)ₕ-;
R_{F}-(CH(CH₃)CH₂O)ₕ-;
R_{F}(-CH₂-CH₂-S)ₕ-,
worin h 1 bis 12 ist; und
worin R_{F} einen fluorhaltigen Bestandteil mit einer der folgenden Strukturen bedeutet:
(a) F(CF₂)ᵢ-, worin i 2 bis 12 ist,
(b) (CF₃)₂CF(CF₂)ⱼ-, worin j 0 bis 8 ist,
(c) R_{F}1(CF₂CF(CF₃))ₖ-, worin k 1 bis 4 ist, und R_{F}1 CF₃-, C₂F₅- oder (CF₃)₂CF-, bedeutet
(d) R_{F}2(R_{F}3)CFO(CF₂CF₂)ₗ-, worin l 1 bis 6 ist und worin R_{F}2 und R_{F}3 unabhängig voneinander jeweils CF₃-, C₂F₅-, n-C₃F₇- oder CF₃CF₂CF(CF₃)- bedeutet oder R_{F}2 und R_{F}3 zusammengenommen -(CF₂)₄- oder -(CF₂)₅ bedeuten, oder
(e) eine der Strukturen (a) bis (d), in denen eines oder mehrere der Fluoratome durch ein oder mehrere Wasserstoff- oder Bromatome und/oder mindestens 2 Chloratome in einem solchen Verhältnis, daß mindestens 50 % der an das Kohlenstoffgerüst von R_{F} gebundenen Atome Fluoratome sind, ersetzt sind, und worin R_{F} mindestens 4 Fluoratome enthält,
m 0 oder 1 ist;
R² R¹, Wasserstoff oder eine OR-Gruppe bedeutet,
worin R ein gesättigtes oder ungesättigtes C₁-C₂₀-Alkyl oder C₃-C₂₀-Acyl bedeutet; und
wenn m 1 ist, R¹ und R² ihre Positionen austauschen können;
und
X und Y unabhängig voneinander jeweils bedeuten:
Hydroxyl;
-OCH₂CH(OH)CH₂OH;
-O(CH₂CH₂O)ₙR³,
worin n eine ganze Zahl von 1 bis 5 ist; und R³ ein Wasserstoffatom oder C₁-C₄-Alkylgruppe bedeutet;
-NR⁴R⁵ oder N⁺R⁴R⁵R⁸,
worin R⁴, R⁵ und R⁸ unabhängig voneinander jeweils ein Wasserstoffatom; eine C₁-C₄-Alkylgruppe;
-CH₂CH₂O(CH₂CH₂O)ₛR³, worin s eine ganze Zahl von 1 bis 5 bedeutet, bedeuten oder R⁴ und R⁵ zusammengenommen -(CH₂)_{q} bedeuten, worin q eine ganze Zahl von 2 bis 5 ist, oder mit dem Stickstoffatom R⁴ und R⁵ eine Morpholinogruppe bilden;
-O(CH₂)ₚZ, worin Z eine 2-Aminoessigsäuregruppe, -NR⁴R⁵ oder -N⁺R⁴R⁵R⁸ bedeutet, worin R⁸ wie für R⁴ und R⁵ oben definiert ist, und p eine ganze Zahl von 1 bis 5 ist;
mit der Maßgabe, daß X und Y nicht beide Hydroxyl oder eine von Hydroxyl abgeleitete ionisierte Form bedeuten.

2. Verbindung nach Anspruch 1, worin R_{F} F(CF₂)ᵢ und m 0 ist.

3. Verbindung nach Anspruch 1, worin R_{F} F(CF₂)ᵢ und m 1 ist.

4. Verbindung nach Anspruch 3, worin R² das gleiche wie R¹ ist.

5. Verbindung nach einem der Ansprüche 1 bis 4, worin R¹ R_{F}(CH₂)ₐ-(CH=CH)_{b}-(CH₂)_{c}-(CH=CH)_{d}-(CH₂)ₑ-A- ist.

6. Verbindung nach Anspruch 5, worin b+d=0.

7. Verbindung nach Anspruch 5, worin A -O-, -C(O)O-, oder -(CH₂)ₙ- bedeutet, worin n=0 oder 1.

8. Verbindung nach einem der Ansprüche 1 bis 4, worin R_{F} F(CF₂)ᵢ- ist und worin i 2 bis 12 ist.

9. Verbindung nach Anspruch 8, worin i 4 bis 8 ist.

10. Verbindung nach einem der Ansprüche 1 bis 4, worin X und Y jeweils unabhängig voneinander Hydroxyl, Morpholino oder eine -O(CH₂CH₂O)ₙR³-Gruppe bedeutet, worin n 1 oder 2 ist und R³ eine Methylgruppe bedeutet, mit der Maßgabe, daß X und Y nicht beide Hydroxyl oder eine von Hydroxyl abgeleitete ionisierte Form bedeuten.

11. Verbindung nach einem der Ansprüche 1 bis 4, worin X und Y jeweils unabhängig voneinander -OCH₂CH₂N⁺(CH₃)₃ bedeutet.

12. Verfahren zur Herstellung einer Verbindung mit der in Anspruch 1 angegebenen Struktur, umfassend die Schritte:
(a) Umsetzen einer Verbindung der allgemeinen Formel 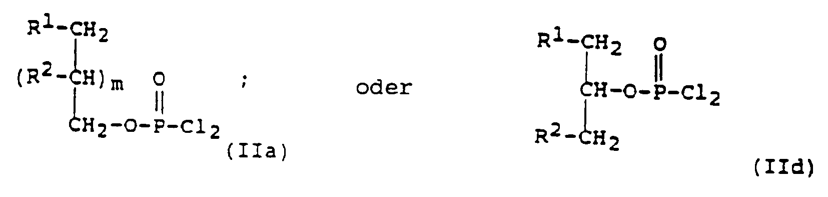 mit einer Verbindung HX, um die Monosubstitution von einem der Cl-Atome zu bewirken, und Umwandeln des monochlorierten Produktes in ein monohydroxyliertes Produkt,
worin X bedeutet:
Hydroxyl;
-OCH₂CH(OH)CH₂OH;
-O(CH₂CH₂O)ₙR³,
worin n eine ganze Zahl von 1 bis 5 ist, und
R³ ein Wasserstoffatom oder C₁-C₄-Alkylgruppe bedeutet;
-NR⁴R⁵ oder N⁺R⁴R⁵R⁸,
worin R⁴, R⁵ und R⁸ jeweils unabhängig voneinander ein Wasserstoffatom, eine C₁-C₄-Alkylgruppe,
-CH₂CH₂O(CH₂CH₂O)ₛR³, worin s eine ganze Zahl von 1 bis 5 bedeutet, bedeuten oder R⁴ und R⁵ zusammen genommen -(CH₂)_{q} bedeuten, worin q eine ganze Zahl von 2 bis 5 ist, oder mit dem Stickstoffatom R⁴ und R⁵ eine Morpholinogruppe bilden;
-O(CH₂)ₚZ
worin Z entweder eine Abgangsgruppe, eine 2-Aminoessigsäure-Gruppe, -NR⁴R⁵ oder -N⁺R⁴R⁵R⁸ bedeutet, worin R⁸ wie für R⁴ und R⁵ oben definiert ist, und p eine ganze Zahl von 1 bis 5 ist.

13. Verfahren nach Anspruch 12, welches ferner den Schritt des Reagierenlassens des Produkts mit HY umfaßt, um eine Disubstitution zu bewirken, worin Y unabhängig die Gruppen wie für X oben definiert bedeutet,
mit der Maßgabe, daß X und Y nicht beide Hydroxyl bedeuten.

14. Verfahren nach Anspruch 12 oder 13, worin X und/oder Y eine -O(CH₂)ₚZ-Gruppe bedeutet, worin p eine ganze Zahl von 1 bis 5 ist und Z eine Abgangsgruppe L bedeutet, umfassend den zusätzlichen Schritt des Umsetzens des mono- oder di-substituierten Produktes mit einer Verbindung HNR⁴R⁵ oder NR⁴R⁵R⁸.

15. Verfahren nach Anspruch 12 oder 13, umfassend den zusätzlichen Schritt des Ersetzens der X- oder Y-Gruppe des Produkts mit einer anderen X- oder Y-Gruppe.

16. Eine stabile, nicht-hämolytische liposomale Formulierung, die eine der Verbindungen der Ansprüche 1 bis 4 umfaßt.

17. Liposomale Formulierung, umfassend eine Verbindung nach einem der Ansprüche 1 bis 4, zusammen mit einem therapeutischen, kosmetischen oder diagnostischen Mittel.

18. Formulierung nach Anspruch 17, worin das Mittel ein Medikament oder Sauerstoff ist.

19. Emulsion, umfassend eine ölige Phase, eine wäßrige Phase und ein oberflächenaktives Mittel, worin das oberflächenaktive Mittel eine Verbindung nach einem der Ansprüche 1 bis 4 ist.

20. Emulsion nach Anspruch 19, worin die ölige Phase einen Fluorkohlenstoff umfaßt.

21. Emulsion nach Anspruch 20, worin der Fluorkohlenstoff hochfluoriert oder perfluoriert ist, und in der Emulsion in einer Konzentration von 10 % bis 70 % Vol/Vol. vorhanden ist.

22. Emulsion nach Anspruch 21, worin die hochfluorierte oder perfluorierte Verbindung eine Molekülmasse von 400 bis 700 hat und ein Bis(F-alkyl)-1,2-ethen, Perfluordecalin, Perfluor-methyldecalin, Perfluor-dimethyldecalin, Perfluormono- oder -dimethyladamantan, Perfluor-trimethylbicyclo-[3.3.1]nonan oder Homologes davon, ein Ether mit der Formel
(CF₃)CFO(CF₂CF₂)OCF(CF₃)₂,
(CF₃)₂CFO(CF₂CF₂)₃OCF(CF₃)₂,
(CF₃)₂CFO(CF₂CF₂)₂F, (CF₃)₂CFO(CF₂CF₂)₃F,
F(CF(CF₃)CF₂O)₂CHFCF₃ oder (C₆F₁₃)₂O,
ein Amin, welches N(C₃F₇)₃, N(C₄F₉)₃ ist,
ein Perfluormethyl-chinolidin oder Perfluorisochinolidin, oder ein Halogenderivat C₆F₁₃Br, C₈F₁₇Br, C₆F₁₃CBr₂CH₂Br, 1-Bromheptadecafluor-4-isopropylcyclohexan oder gemischte Kohlenwasserstoff/Fluorkohlenstoffverbindungen mit einer niedrigeren Molekülmasse, CₙF₂ₙ₊₁CₘN₂ₘ₊₁, CₙF₂ₙ₊₁CₘH₂ₘ₋₁,in denen die Kohlenwasserstoffkette eine Doppelbindung enthält, worin n eine ganze Zahl zwischen 1 und 10 und m eine ganze Zahl zwischen 1 und 20 ist, oder Analoge oder Gemische davon ist.

23. Emulsion nach Anspruch 21, worin das Bis(F-alkyl)-1,2-ethen ein Bis(F-butyl)-1,2-ethen, ein F-Isopropyl-1- F-Hexyl-2-ethen oder ein Bis(F-hexyl)-1,2-ethen ist.

24. Verbindung mit der allgemeinen Struktur: 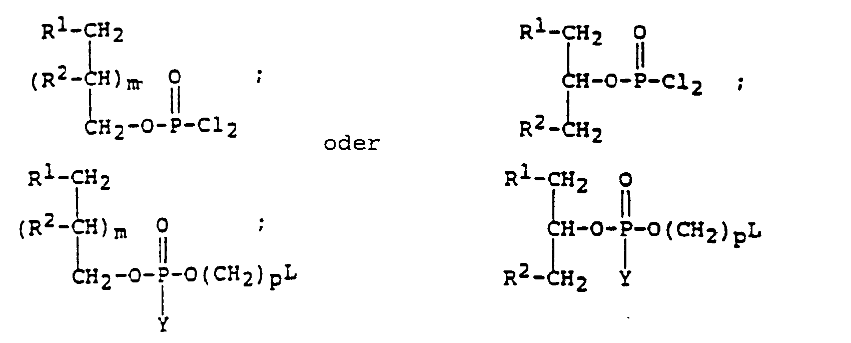 worin R¹, R², Y, m und p die gleiche Bedeutung wie in Anspruch 1 haben; und L entweder Z mit der gleichen Bedeutung wie in Anspruch 1 oder eine Abgangsgruppe ist.

## Revendications

1. Composé représenté par la formule générale : 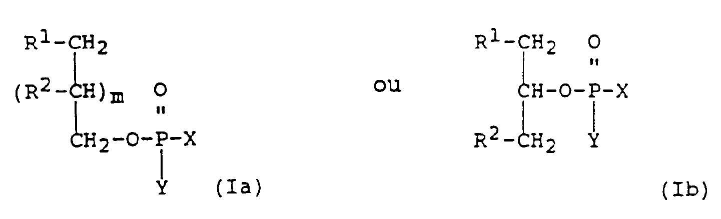 dans laquelle :
- R¹ représente :
R_{F}(CH₂)ₐ-(CH=CH)_{b}-(CH₂)_{c}-(CH=CH)_{d}-(CH₂)ₑ-A- ;
R_{F}-(CH₂)_{f}-OCH₂CH(CH₂OH)CH₂-A- ;
R_{F}-(CH₂)_{g}-OCH₂CH(CH₂OH)-A- ;
où -A- représente -O-, -C(O)O-, -R⁶(R⁷)N⁺-, (où R⁶ et R⁷ représentent chacun alkyle en C₁-C₄ ou hydroxyéthyle), -(CH₂)ₙ-, où n = 0 ou 1, ou -C(O)N(R⁹)-(CH₂)_{q}-B, où q est un nombre entier de 0 à 12, B représente -O- ou -C(O)-, et R⁹ représente hydrogène ou R⁶,
et où la somme de a+c+e a une valeur de 0 à 11, la somme b+d a une valeur de 0 à 12, et f et g ont chacun une valeur de 1 à 12 ;
R_{F}-(CH₂-CH₂-O)ₕ- ,
R_{F}-(CH(CH₃)CH₂O)ₕ- ;
R_{F}(-CH₂-CH₂-S)h-,
où h a une valeur de 1 à 12 ; et
où R_{F} représente une fraction contenant du fluor, ayant l'une des structures suivantes :
(a) F(CF₂)ᵢ-, où i a une valeur de 2 à 12,
(b) (CF₃)₂CF(CF₂)ⱼ-, où j a une valeur de 0 à 8,
(c) R_{F}1(CF₂CF(CF₃))ₖ-, où k a une valeur de 1 à 4, et R_{F}1 représente CF₃-, C₂F₅- ou (CF₃)₂CF-,
(d) R_{F}2(R_{F}3)CFO(CF₂CF₂)ₗ-, où l a une valeur de 1 à 6 et où R_{F}2 et R_{F}3 représentent chacun indépendamment CF₃-, C₂F₅-, n-C₃F₇- ou CF₃CF₂CF(CF₃)-, ou bien R_{F}2 et R_{F}3, pris ensemble, représentent -(CF₂)₄- ou -(CF₂)₅-, ou
(e) l'une des structures (a) à (d), dans laquelle un ou plusieurs des atomes de fluor sont substitués par un ou plusieurs atomes d'hydrogène ou de brome et/ou au moins deux atomes de chlore dans une proportion telle qu'au moins 50% des atomes liés au squelette carboné de RF soient des atomes de fluor, et où RF contient au moins 4 atomes de fluor,
- m vaut 0 ou 1 ;
- R² représente R¹, hydrogène ou un groupe OR,
où R représente un alkyle en C₁-C₂₀ ou acyle en C₃-C₂₀, saturé ou insaturé ; et
lorsque m vaut 1, R¹ et R² peuvent échanger leurs positions ; et
- X et Y représentent chacun indépendamment :
hydroxyle ;
-OCH₂CH(OH)CH₂OH ;
-O(CH₂CH₂O)ₙR³,
où n est un nombre entier de 1 à 5 ; et
R³ représente un atome d'hydrogène ou un groupe alkyle en C₁-C₄ ;
-NR⁴R⁵ ou N⁺R⁴R⁵R⁸,
où R⁴, R⁵ et R⁸ représentent chacun indépendamment un atome d'hydrogène ; un groupe alkyle en C₁-C₄ ; -CH₂CH₂O(CH₂CH₂O)ₛR³, où s représente un nombre entier de 1 à 5, ou bien R⁴ et R⁵, lorsqu'ils sont pris ensemble, représentent -(CH₂)_{q}, où q est un nombre entier de 2 à 5, ou bien avec l'atome d'azote, R⁴ et R⁵ forment un groupe morpholino ;
-O(CH₂)ₚZ,
où Z représente un groupe acide amino-2 acétique, -NR⁴R⁵ ou -N⁺R⁴R⁵R⁸, où R⁸ est tel que défini pour R⁴ et R⁵ ci-dessus, et p est un nombre entier de 1 à 5 ;
avec la condition que X et Y ne représentent pas tous les deux hydroxyle ou une forme ionisée issue d'hydroxyle.

2. Composé selon la revendication 1, dans lequel R_{F} représente F(CF₂)ᵢ et m vaut 0.

3. Composé selon la revendication 1, dans lequel R_{F} représente F(CF₂)ᵢ et m vaut 1.

4. Composé selon la revendication 3, dans lequel R² est identique à R¹.

5. Composé selon l'une des revendications 1 à 4, dans lequel R¹ représente
R_{F}(CH₂)ₐ-(CH=CH)_{b}-(CH₂)_{c}-(CH=CH)_{d}-(CH₂)ₑ-A-.

6. Composé selon la revendication 5, dans lequel b+d = 0.

7. Composé selon la revendication 5, dans lequel A représente -O-, -C(O)O- ou -(CH₂)ₙ-, où n = 0 ou 1.

8. Composé selon l'une des revendications 1 à 4, dans lequel R_{F} représente F(CF₂)ᵢ-, et dans lequel i a une valeur de 2 à 12.

9. Composé selon la revendication 8, dans lequel i a une valeur de 4 à 8.

10. Composé selon l'une des revendications 1 à 4, dans lequel X et Y représentent chacun indépendamment hydroxyle, morpholino, ou un groupe -O(CH₂CH₂O)ₙR³, où n vaut 1 ou 2 et R³ représente un groupe méthyle, avec la condition que X et Y ne représentent pas tous les deux hydroxyle ou une forme ionisée issue d'hydroxyle.

11. Composé selon l'une des revendications 1 à 4, dans lequel X et Y représentent chacun indépendamment -OCH₂CH₂N⁺(CH₃)₃.

12. Procédé de fabrication d'un composé ayant la structure telle que définie à la revendication 1, comprenant les étapes consistant à :
(a) faire réagir un composé représenté par la formule générale : 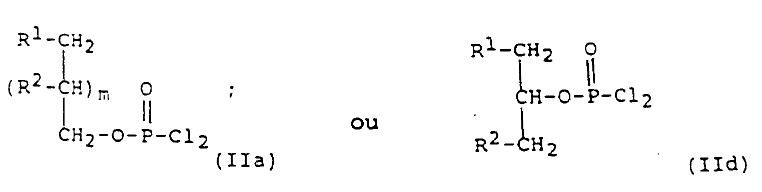 avec un composé HX, afin d'effectuer la mono-substitution de l'un des atomes de Cl, et la conversion du produit mono-chloré en un produit mono-hydroxylé,
où X représente :
hydroxyle ;
-OCH₂CH(OH)CH₂OH ;
-O(CH₂CH₂O)ₙR³
où n est un nombre entier de 1 à 5 ; et
R³ représente un atome d'hydrogène ou un groupe alkyle en C₁-C₄ ;
-NR⁴R⁵ ou N⁺R⁴R⁵R⁸,
où R⁴, R⁵ et R⁸ représentent chacun indépendamment un atome d'hydrogène ; un groupe alkyle en C₁-C₄ ; -CH₂CH₂O(CH₂CH₂O)ₛR³, où s représente un nombre entier de 1 à 5, ou bien R⁴ et R⁵, lorsqu'ils sont pris ensemble, représentent -(CH₂)_{q}, où q est un nombre entier de 2 à 5, ou bien, avec l'atome d'azote, R⁴ et R⁵ forment un groupe morpholino ;
-O(CH₂)ₚZ,
où Z représente l'un parmi un groupe partant, un groupe acide amino-2 acétique, -NR⁴R⁵ ou -N⁺R⁴R⁵R⁸, où R⁸ est tel que défini pour R⁴ et R⁵ ci-dessus, et p est un nombre entier de 1 à 5.

13. Procédé selon la revendication 12, comprenant en outre l'étape consistant à faire réagir le produit avec HY, pour effectuer une disubstitution, où Y représente indépendamment les groupes tels que définis pour X ci-dessus, avec la condition que X et Y ne représentent pas tous les deux hydroxyle.

14. Procédé selon la revendication 12 ou 13, dans lequel X et/ou Y représente(nt) un groupe -O(CH₂)ₚZ, où p est un nombre entier de 1 à 5 et Z représente un groupe partant, L, comprenant l'étape supplémentaire consistant à faire réagir ledit produit mono- ou di-substitué avec un composé HNR⁴R⁵ ou NR⁴R⁵R⁸.

15. Procédé selon la revendication 12 ou 13, comprenant l'étape supplémentaire consistant à remplacer ledit groupe X ou Y du produit par un groupe X ou Y différent.

16. Formulation de liposomes, non-hémolytique, stable, comprenant l'un des composés tels que définis à l'une des revendications 1 à 4.

17. Formulation de liposomes comprenant un composé tel que défini à l'une des revendications 1 à 4, conjointement avec un agent thérapeutique, cosmétique ou de diagnostic.

18. Formulation selon la revendication 17, dans laquelle ledit agent est un médicament ou l'oxygène.

19. Emulsion comprenant une phase huileuse, une phase aqueuse et un agent tensio-actif, dans laquelle ledit agent tensio-actif est un composé tel que défini à l'une des revendications 1 à 4.

20. Emulsion selon la revendication 19, dans laquelle la phase huileuse comprend un fluorocarbure.

21. Emulsion selon la revendication 20, dans laquelle le fluorocarbure est fortement fluoré ou perfluoré, et est présent dans ladite émulsion à une concentration de 10% à 70% en volume/volume.

22. Emulsion selon la revendication 21, dans laquelle le composé fortement fluoré ou perfluoré présente une masse moléculaire de 400 à 700 et est un bis (F-alkyl)-1,2-éthène, la perfluorodécaline, la perfluoro-méthyldécaline, la perfluoro-diméthyldécaline, le perfluoromono- ou diméthyladamantane, le perfluoro-triméthylbicyclo-/3,3,1/nonane ou un homologue de ces composés, un éther représenté par la formule (CF₃)CFO(CF₂CF₂)OCF(CF₃)₂, (CF₃)₂CFO(CF₂CF₂)₃OCF(CF₃)₂, (CF₃)₂CFO(CF₂CF₂)₂F, (CF₃)₂CFO(CF₂CF₂)₃F, F(CF(CF₃)CF₂O)₂CHFCF₃, ou (C₆F₁₃)₂O, une amine qui est N(C₃F₇)₃, N(C₄F₉)₃, la perfluorométhylquinolidine ou la perfluoroisoquinolidine, ou un dérivé halogéné C₆F₁₃Br, C₈F₁₇Br, C₆F₁₃CBr₂CH₂Br, le bromo-1 heptadécafluoro-isopropyl-4 cyclohexane ou des composés mixtes hydrocarbure/ fluorocarbure ayant une masse moléculaire inférieure, CₙF₂ₙ₊₁CₘN₂ₘ₊₁, CₙF₂ₙ₊₁CₘH₂ₘ₋₁, dans lesquels la chaîne hydrocarbonée contient une double liaison, où n est un nombre entier entre 1 et 10 et m est un nombre entier entre 1 et 20, ou des analogues ou mélanges de ces composés.

23. Emulsion selon la revendication 21, dans laquelle ledit bis(F-alkyl)-1,2-éthène est un bis(F-butyl)-1,2-éthène, un F-isopropyl-1, F-hexyl-2-éthène, ou un bis(F-hexyl)-1,2-éthène.

24. Composé représenté par la structure générale: 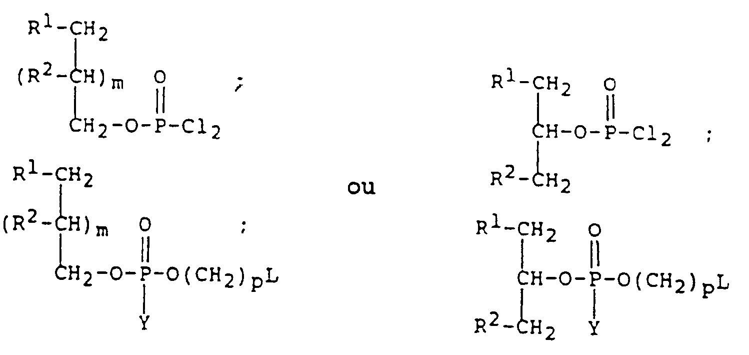 dans laquelle :
- R¹, R², Y, m et p ont les mêmes significations qu'à la revendication 1 ; et
- L représente soit Z ayant la même signification qu'à la revendication 1, soit un groupe partant.
